# EUROPEAN PATENT APPLICATION

(11) **EP 4 491 188 A1**
(43) Date of publication of application: **15.01.2025**
(21) Application number: 23766984.1
(22) Date of filing: 10.03.2023
(51) Int. Cl.: A61K 35/12, A61K 38/18, A61K 47/42, A61P 25/28

(54) **THERAPEUTIC AGENT FOR SPINAL CORD INJURY**

(30) Priority: 11.03.2022 WO PCT/JP2022/010976
(71) Applicant: Keio University, Tokyo, 108-8345 (JP); Kringle Pharma, Inc., Ibaraki-shi, Osaka 567-0085 (JP)
(72) Inventor: HASHIMOTO, Shogo, Tokyo 160-8582 (JP); NAGOSHI, Narihito, Tokyo 160-8582 (JP); NAKAMURA, Masaya, Tokyo 160-8582 (JP); OKANO, Hideyuki, Tokyo 160-8582 (JP)
(74) Representative: Krauss, Jan
(86) International application number: PCT/JP2023/009421
(87) International publication number: WO 2023/171808

(57) **Abstract**

Provided is a spinal cord injury therapeutic agent for chronic incomplete spinal cord injury or chronic complete spinal cord injury that has heretofore been difficult to treat. The inventors of the present invention have recognized that an agent, which includes (a) a hepatocyte growth factor protein, a carrier configured to carry, and to be capable of sustained-releasing, the (a), and an iPS cell-derived neural stem and/or progenitor cells, has a spinal cord injury-treating effect, a motor function-improving effect, and a voiding function-improving effect in the chronic incomplete spinal cord injury or the chronic complete spinal cord injury. Thus, the inventors have completed the present invention.

## Description

### Technical Field

The present invention relates to a therapeutic agent for spinal cord injury, in particular, a therapeutic agent for chronic incomplete spinal cord injury or chronic complete spinal cord injury. The present invention also relates to an agent for alleviating motor dysfunction, an agent for alleviating voiding dysfunction, a lower limb muscle mass-increasing agent, a spinal cord cavitation inhibitor, an anti-inflammatory agent for a lesion site, an angiogenesis promoter, a scar formation inhibitor for the lesion site, and an axon extension promoter for the lesion site in the chronic incomplete spinal cord injury or the chronic complete spinal cord injury.

The respective agents of the present invention are sometimes collectively referred to as "agents of the present invention."

In addition, the present application claims priority from PCT/JP2022/10976, the disclosure of which is incorporated herein by reference.

### Background Art

### (Spinal Cord Injury)

Spinal cord injury is a disease state showing the paralysis of sensory, motor, and autonomic nervous systems below a lesion with the injury of a spinal cord parenchyma by a trauma or the like as a trigger, and the total number of the patients of the injury in Japan has reached 150,000 or more. Further, about 5,000 new patients are occurring every year.

However, it has been considered that the central nervous system of a mammal is not regenerated once the system is injured, and hence under the current circumstances, no effective therapeutic method has been established yet.

The disease state of the spinal cord injury varies depending on a timing from the traumatism of a spinal cord, and particularly in its chronic phase, a nerve fiber in a lesion of the spinal cord falls, and nerve regeneration is inhibited by a glial scar formed in or around a cavity. Accordingly, the degree of difficulty of a therapeutic method for the injury is extremely high. Further, in a spinal cord that has been completely transected called complete injury, it has been considered difficult to perform functional recovery through nerve regeneration because there are no nerve fibers intersecting each other in the lesion.

Although investigations on acute or subacute incomplete spinal cord injury have been vigorously performed worldwide, no effective therapeutic methods in chronic incomplete spinal cord injury and chronic complete spinal cord injury accounting for a large majority of spinal cord injury patients have been reported, and hence the development of therapeutic methods for the chronic incomplete spinal cord injury and the chronic complete spinal cord injury is urgent.

### (Hepatocyte Growth Factor)

A hepatocyte growth factor (hereinafter sometimes abbreviated as "HGF" in this description) protein was discovered as a bioactive protein having growth-promoting activity on a mature hepatocyte. As a result of subsequent investigations, it has been known that the HGF protein acts on the c-Met receptors of many cells, such as an epithelial cell and a vascular endothelial cell, as well as a hepatocyte, to be involved in the damage repair and regeneration of tissues and organs. The HGF protein can be produced in a large amount as a recombinant protein by a bioengineering approach, and the recombinant HGF protein has been expected to be applied as a therapeutic agent not only for hepatitis and liver cirrhosis but also for a renal disease, a wound, or the like.

Meanwhile, many investigations in the expression and functional analysis of a gene including a recent knock-out/knock-in mouse approach have revealed that the HGF protein also has promoting actions on the survival of a nerve cell and the elongation of a neurite, and is hence a factor important as a neurotrophic factor.

The HGF protein shows neurotrophic factor activity on each of nerve cells, such as a hippocampal neuron, a dopaminergic neuron, a cerebellar granule cell, a sensory neuron, and a motor neuron. The HGF protein shows a strong survival-promoting action particularly on the motor neuron, and the activity is comparable to the activity of a glial cell line-derived neurotrophic factor (GDNF) known to have the strongest survival-promoting action on the motor neuron.

It has been reported that the HGF protein may be used as a therapeutic agent for various nerve diseases including amyotrophic lateral sclerosis (ALS) and spinal cord injury on the basis of such neurotrophic activity (see Patent Literature 1).

### (Prior Non Patent Literature)

In Non Patent Literature 1, there is a disclosure of the effectiveness of acute spinal cord injury, but there is also a disclosure that "the transplantation of a cell to chronic spinal cord injury alone cannot provide an improvement in motor function."

In Non Patent Literature 2, there is a disclosure "that the administration of a hepatocyte growth factor and an iPS cell-derived NSC after spinal cord injury is assumed to be effective in regeneration after the spinal cord injury." However, there is no disclosure or suggestion of the configuration of each of the agents of the present invention.

In Non Patent Literature 3, there is a disclosure of a "therapeutic method including applying a gelatin-furfurylamine (FA) hydrogel and a CBD-HGF or a HGF in combination to an animal whose spinal cord has been injured by compression." However, in Non Patent Literature 3, there is a disclosure of a verification in an acute spinal cord injury model, and reference is made to the fact that no satisfactory recovery is achieved. Further, in Non Patent Literature 3, there is no disclosure or suggestion of the configuration of each of the agents of the present invention.

In Non Patent Literature 4, there is a description "that the combination of two elements, that is, iPS-derived neural stem cell transplantation and a HGF is expected to exhibit a higher therapeutic effect." However, there is no disclosure or suggestion of a specific configuration or method.

In Patent Literature 2, there is a disclosure of a "columnar HGF protein-containing sustained release preparation obtained by mixing an aqueous solution of a HGF protein and a solution of atelocollagen in a phosphate buffer, then freeze-drying the mixture, and subjecting the dried product to compression molding." However, there is no disclosure or suggestion of the configuration of each of the agents of the present invention.

In Patent Literature 3, there is a disclosure of a "therapeutic agent for nerve injury including a differentiated cell-derived pluripotent stem cell." However, there is no disclosure or suggestion of the configuration of each of the agents of the present invention.

### Citation List

### Patent Literature

[PTL 1] JP 2018-44000 A
[PTL 2] JP 5419045 B2
[PTL 3] JP 2009-215191 A

### Non Patent Literature

[NPL 1] Molecular Brain volume 6, Article number: 3 (2013)
[NPL 2] Int. J. Mol. Sci. 2019, 20(15), 3838
[NPL 3] Sci. Rep. 2018, 8: 917.
[NPL 4] Medical Plaza, 2020, vol. 60, No. 9, pp. 4-6

### Summary of Invention

### Technical Problem

An object of the present invention is to provide a therapeutic agent for spinal cord injury, in particular, chronic incomplete spinal cord injury or chronic complete spinal cord injury that has heretofore been difficult to treat.

### Solution to Problem

The inventors of the present invention have recognized that an agent including the following (1) to (3) has a spinal cord injury-treating effect on chronic incomplete spinal cord injury or chronic complete spinal cord injury, a motor dysfunction-alleviating effect, and a voiding dysfunction-alleviating effect, and thus, the inventors have completed the present invention:
(1) a HGF protein;
(2) a carrier configured to carry, and to be capable of sustained-releasing, the HGF protein; and
(3) an iPS cell-derived neural stem and/or progenitor cells.

The present invention includes the following.
1. A therapeutic agent for treatment of chronic incomplete or chronic complete spinal cord injury, including the following:
   (1) (a) a hepatocyte growth factor protein or a substance having a c-Met phosphorylation action comparable to that of the hepatocyte growth factor protein, or (b) a gene encoding the hepatocyte growth factor protein or a gene encoding the substance having a c-Met phosphorylation action comparable to that of the hepatocyte growth factor protein;
   (2) a carrier configured to carry, and to be capable of sustained-releasing, the (a) or the (b); and
   (3) a pluripotent stem cell,
      wherein the carrier is implanted in a lesion site of a spinal cord, and the pluripotent stem cells are administered to the carrier after the implantation or to the lesion site of the spinal cord.
2. The therapeutic agent according to Item 1, wherein the pluripotent stem cells are an iPS cell-derived neural stem and/or progenitor cells.
3. The therapeutic agent according to Item 1 or 2, wherein the pluripotent stem cells are administered to the carrier after the implantation.
4. The therapeutic agent according to Item 1, wherein the substance having a c-Met phosphorylation action is a hepatocyte growth factor protein, and the pluripotent stem cells are an iPS cell-derived neural stem and/or progenitor cells.
5. The therapeutic agent according to any one of Items 1 to 4, wherein the treatment is to promote regeneration of an injured spinal cord and/or to alleviate an accessory symptom accompanying the spinal cord injury.
6. The therapeutic agent according to Item 5, wherein the promotion of the regeneration of the injured spinal cord is promotion of axon extension, an increase in number of nerve fibers, neurogenesis of an endogenous stem cell, angiogenesis, inhibition of scar formation, inhibition of inflammation, angiogenesis, and/or inhibition of spinal cord cavitation.
7. The therapeutic agent according to Item 5, wherein the accessory symptom accompanying the injured spinal cord is motor dysfunction and/or voiding dysfunction.
8. The therapeutic agent according to Item 7, wherein the motor dysfunction is motor dysfunction of a lower limb.
9. The therapeutic agent according to Item 7, wherein the motor dysfunction is due to a reduction in muscle mass.
10. An agent for alleviating voiding dysfunction in chronic incomplete spinal cord injury or chronic complete spinal cord injury, including the following:
   (1) a hepatocyte growth factor protein, and a carrier configured to carry, and to be capable of sustained-releasing, the hepatocyte growth factor protein; and
   (2) an iPS cell-derived neural stem and/or progenitor cells,
      wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.
11. The therapeutic agent according to Item 10, wherein the iPS cell-derived neural stem and/or progenitor cells are administered to the carrier after the implantation.
12. A therapeutic agent for chronic incomplete or chronic complete spinal cord injury, including:
   a hepatocyte growth factor protein; and
   a carrier configured to carry, and to be capable of sustained-releasing, the hepatocyte growth factor protein,
   wherein the carrier carrying the hepatocyte growth factor protein is implanted in a lesion site of a spinal cord, and an iPS cell-derived neural stem and/or progenitor cells are administered to the carrier after the implantation or to the lesion site of the spinal cord.
13. A therapeutic agent for chronic incomplete or chronic complete spinal cord injury, including an iPS cell-derived neural stem and/or progenitor cells,
   wherein the iPS cell-derived neural stem and/or progenitor cells is administered to a lesion site of a spinal cord or a carrier implanted in the lesion site of the spinal cord, the carrier carrying a hepatocyte growth factor protein.
14. An agent for alleviating motor dysfunction in chronic incomplete spinal cord injury or chronic complete spinal cord injury, including the following:
   (1) a hepatocyte growth factor protein, and a carrier configured to carry, and to be capable of sustained-releasing, the hepatocyte growth factor protein; and
   (2) an iPS cell-derived neural stem and/or progenitor cells,
      wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.
15. The agent for alleviating motor dysfunction according to Item 14, wherein the motor dysfunction is motor dysfunction of a lower limb.
16. A lower limb muscle mass-increasing agent in chronic incomplete spinal cord injury or chronic complete spinal cord injury, including the following:
   (1) a hepatocyte growth factor protein, and a carrier configured to carry, and to be capable of sustained-releasing, the hepatocyte growth factor protein; and
   (2) an iPS cell-derived neural stem and/or progenitor cells,
      wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.
17. A spinal cord cavitation inhibitor in chronic incomplete spinal cord injury or chronic complete spinal cord injury, including the following:
   (1) a hepatocyte growth factor protein, and a carrier configured to carry, and to be capable of sustained-releasing, the hepatocyte growth factor protein; and
   (2) an iPS cell-derived neural stem and/or progenitor cells,
      wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.
18. An anti-inflammatory agent for a lesion site in chronic incomplete spinal cord injury or chronic complete spinal cord injury, including the following:
   (1) a hepatocyte growth factor protein, and a carrier configured to carry, and to be capable of sustained-releasing, the hepatocyte growth factor protein; and
   (2) an iPS cell-derived neural stem and/or progenitor cell,
      wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.
19. An angiogenesis promoter for a lesion site in chronic incomplete spinal cord injury or chronic complete spinal cord injury, including the following:
   (1) a hepatocyte growth factor protein, and a carrier configured to carry, and to be capable of sustained-releasing, the hepatocyte growth factor protein; and
   (2) an iPS cell-derived neural stem and/or progenitor cell,
      wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.
20. A scar formation inhibitor for a lesion site in chronic incomplete spinal cord injury or chronic complete spinal cord injury, including the following:
   (1) a hepatocyte growth factor protein, and a carrier configured to carry, and to be capable of sustained-releasing, the hepatocyte growth factor protein; and
   (2) an iPS cell-derived neural stem and/or progenitor cell,
      wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.
21. An axon extension promoter for a lesion site in chronic incomplete spinal cord injury or chronic complete spinal cord injury, including the following:
   (1) a hepatocyte growth factor protein, and a carrier configured to carry, and to be capable of sustained-releasing, the hepatocyte growth factor protein; and
   (2) an iPS cell-derived neural stem and/or progenitor cells,
      wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.
22. A therapeutic method for chronic incomplete or chronic complete spinal cord injury, including the following steps:
   (1) a step of implanting, in a lesion site of a spinal cord of a target having spinal cord injury, a carrier configured to carry, and to be capable of sustained-releasing,
      (a) a hepatocyte growth factor protein or a substance having a c-Met phosphorylation action comparable to that of the hepatocyte growth factor protein, or
      (b) a gene encoding the hepatocyte growth factor protein or a gene encoding the substance having a c-Met phosphorylation action comparable to that of the hepatocyte growth factor protein; and
   (2) a step of administering a pluripotent stem cell to the carrier after the implantation or to the lesion site of the spinal cord.
23. A kit for treating chronic incomplete or chronic complete spinal cord injury, including the following:
   (1) (a) a hepatocyte growth factor protein or a substance having a c-Met phosphorylation action comparable to that of the hepatocyte growth factor protein, or (b) a gene encoding the hepatocyte growth factor protein or a gene encoding the substance having a c-Met phosphorylation action comparable to that of the hepatocyte growth factor protein;
   (2) a carrier configured to carry, and to be capable of sustained-releasing, the (a) or the (b); and
   (3) a pluripotent stem cell,
      wherein the carrier configured to carry the (a) or the (b) is implanted in a lesion site of a spinal cord of a spinal cord injury patient, and the pluripotent stem cells are administered to the carrier after the implantation or to the lesion site of the spinal cord.

### Advantageous Effects of Invention

The agents of the present invention each have one or more of the following effects:
(1) an inhibiting effect on spinal cord cavitation in a lesion site of a spinal cord;
(2) an increasing effect on the survival and engraftment rate of grafted cells;
(3) an increasing effect on the number of grafted cell-derived nerve fibers;
(4) an increasing effect on the number of host-derived nerve fibers in the lesion site of the spinal cord;
(5) an increasing effect on the number of newborn blood vessels in the lesion site of the spinal cord;
(6) an inhibiting effect on a fibrous scar in the lesion site of the spinal cord;
(7) an inhibiting effect on scar formation in the lesion site of the spinal cord;
(8) an alleviating effect on motor dysfunction due to spinal cord injury;
(9) an alleviating effect on voiding dysfunction due to the spinal cord injury;
(10) a sustained-releasing effect on a HGF protein in the lesion site of the spinal cord;
(11) an anti-inflammatory action and effect in the lesion site of the spinal cord;
(12) a neuroprotective action and effect in the lesion site of the spinal cord;
(13) a promoting effect on axon extension in the lesion site of the spinal cord (in particular, an increasing effect on the number of regenerated axons and an increasing effect on the density of the regenerated axons);
(14) a growing effect on an endogenous neural stem/progenitor cells in the lesion site of the spinal cord; and
(15) an inducing effect on the differentiation of an endogenous neural stem/progenitor cell into a nerve cell in the lesion site of the spinal cord.

### Brief Description of Drawings

FIG. 1 is a view for illustrating a method of evaluating a HGF protein-containing scaffold for a spinal cord microenvironment after chronic complete spinal cord injury. The evaluation was performed in each of three groups, that is, a HGF protein-containing scaffold group, a scaffold only group, and a control group in which no treatment was performed in a chronic phase on a 42nd day after the complete transection of a spinal cord. The spinal cord was collected on a 7th day or a 14th day, and was evaluated by immunostaining and a Western blot.
FIG. 2 is a graph for showing the recognition of a sustained-releasing effect on a HGF protein (in vivo) exhibited by a scaffold.
FIG. 3 is a graph for showing the evaluation of an angiogenesis-promoting action in a lesion epicenter in the HGF protein-containing scaffold (the left figure is a graph for showing the "evaluation of a newborn blood vessel in the lesion epicenter on a 7th day after the implantation of the scaffold," and the right figure is a graph for showing the "evaluation of a newborn blood vessel in the HGF protein-containing scaffold group (on a 7th or 14th day after the implantation)").
FIG. 4 is a graph for showing the evaluation of an anti-inflammatory action exhibited by the HGF protein-containing scaffold (Arginase-1).
FIG. 5 is a graph for showing the evaluation of the anti-inflammatory action exhibited by the HGF protein-containing scaffold (Arginase-1 positive cell/iba1 positive cell).
FIG. 6 is a graph for showing the evaluation of the anti-inflammatory action exhibited by the HGF protein-containing scaffold (TNF-a inhibition).
FIG. 7 is a graph for showing the evaluation of a neuroprotective action exhibited by the HGF protein-containing scaffold (BDNF).
FIG. 8 is a graph for showing the evaluation of the inhibition of scar formation by the HGF protein-containing scaffold (TGF-b inhibition).
FIG. 9 is a graph for showing the evaluation of an axon-regenerating or axon-reextending action exhibited by the HGF protein-containing scaffold (the number of axons (left figure) and the density of the axons (right figure)).
FIG. 10 is a view for illustrating a method of evaluating the activation of an endogenous neural stem cell or progenitor cells by the HGF protein-containing scaffold.
FIG. 11 is a photograph for showing the evaluation of the activation of the endogenous neural stem/progenitor cells by the HGF protein-containing scaffold (immunostaining images of SOX2, Musashi 1, and EdU).
FIG. 12 is a photograph for showing the evaluation of the activation of the endogenous neural stem/progenitor cells by the HGF protein-containing scaffold (immunostaining images of GFAP, Musashi 1, and EdU).
FIG. 13 is a graph for showing the quantitative evaluation of the activation of the endogenous neural stem/progenitor cells by the HGF protein-containing scaffold.
FIG. 14 is an immunostaining image for showing the evaluation of a differentiating action on the endogenous neural stem/progenitor cells into a neuron exhibited by the HGF protein-containing scaffold.
FIG. 15 is a view for illustrating a method of evaluating each of therapeutic agents (agents of the present invention) used in combination with the administration of an iPS cell-derived neural stem/progenitor cells.
FIG. 16 is a photograph for showing a spinal cord finding in a lesion on a 42nd day after the administration of the iPS cell-derived neural stem/progenitor cells.
FIG. 17 is a graph for showing the evaluation of the survival and engraftment rate of the administered iPS cell-derived neural stem/progenitor cells.
FIG. 18 is a graph for showing the evaluation of a grafted cell-derived nerve fiber around a lesion epicenter (mid-sagittal section).
FIG. 19 is a graph for showing the evaluation of a host-derived nerve fiber around the lesion epicenter (mid-sagittal section).
FIG. 20 is a graph for showing the evaluation of a serotonergic neuron around the lesion epicenter (mid-sagittal section).
FIG. 21 is a graph for showing the evaluation of a newborn blood vessel around the lesion epicenter (mid-sagittal section).
FIG. 22 is a graph for showing the evaluation of a fibrous scar and cavity formation (mid-sagittal section).
FIG. 23 is a graph for showing the evaluation of a glial scar (mid-sagittal section).
FIG. 24 is a graph for showing the evaluation of a lower limb motor function (a BBB score, the stride length of a Digigait, and the muscle weight of a lower leg).
FIG. 25 is a graph for showing the evaluation of a motor function (MEP).
FIG. 26 is a photograph or a graph for showing the evaluation of a voiding function (bladder wall).
FIG. 27 is a graph for showing the evaluation of the voiding function (lumbar enlargement).
FIG. 28 is a graph for showing the expression amount of the HGF protein around the epicenter.

### Description of Embodiments

### (Target of the Present Invention)

The targets of the present invention (agents of the present invention) are therapeutic agents for spinal cord injury, in particular, therapeutic agents for chronic incomplete spinal cord injury or therapeutic agents for chronic complete spinal cord injury, and are motor function- and voiding function-improving agents for the spinal cord injury, a lower limb muscle mass-increasing agent in the spinal cord injury, a spinal cord cavitation inhibitor, an anti-inflammatory agent for a lesion site, an angiogenesis promoter, a scar formation inhibitor for the lesion site, and an axon extension promoter for the lesion site.

The agents of the present invention each include the following:
(1) (a) a HGF protein or a substance having a c-Met phosphorylation action comparable to that of the HGF protein, or (b) a gene encoding the HGF protein or a gene encoding the substance having a c-Met phosphorylation action comparable to that of the HGF protein;
(2) a carrier configured to carry, and to be capable of sustained-releasing, the (a) or the (b); and
(3) a pluripotent stem cell.

### (Spinal Cord Injury)

Although the acute phase and subacute phase of spinal cord injury, chronic incomplete spinal cord injury, and chronic complete spinal cord injury may each serve as a target to be treated with each of the agents of the present invention, the agent may be preferably intended for the treatment of the chronic incomplete spinal cord injury or the chronic complete spinal cord injury that has been difficult to treat by a conventional therapeutic method.

The treatment includes an improvement, alleviation, relapse prevention, and complete recovery.

### (Target to be treated)

The targets to be treated with the agents of the present invention are not particularly limited as long as the targets each have a spinal cord. Examples thereof may include a human, a monkey, a cow, a horse, a pig, a sheep, a dog, a cat, a rat, a mouse, a rabbit, a hamster, a guinea pig, and a chimpanzee.

### (HGF Protein and Gene encoding HGF Protein)

The kind of the HGF protein in the present invention is not particularly limited, and for example, HGF proteins derived from various animals (natural HGF proteins or recombinant proteins produced by a genetic engineering technology) may each be suitably used. In the present invention, for example, a HGF protein derived from an animal to which each of the agents of the present invention is applied is preferably used. For example, when each of the agents of the present invention is applied to a human, a human-derived HGF protein (hereinafter sometimes referred to as "human HGF protein") is suitably used as the HGF protein to be used in the present invention.

A recombinant human HGF protein is more preferred.

In addition, the HGF protein to be used in the present invention may be a deletion type (dHGF) from which 5 amino acid residues have been deleted.

The human HGF protein is preferably, for example, a protein to be encoded by DNA formed of a base sequence represented by SEQ ID NO: 1 or SEQ ID NO: 2. More specifically, for example, a protein formed of an amino acid sequence represented by SEQ ID NO: 3, a protein formed of an amino acid sequence represented by SEQ ID NO: 4, a protein formed of an amino acid sequence represented by SEQ ID NO: 5, or a protein formed of an amino acid sequence represented by SEQ ID NO: 6 is preferred.

Of those, a protein having an amino acid sequence represented by SEQ ID NO: 5 or SEQ ID NO: 6 is preferred as the human HGF protein, and a protein formed of an amino acid sequence represented by SEQ ID NO: 5 or SEQ ID NO: 6 is more preferred. For example, a HGF protein formed of the amino acid sequence represented by SEQ ID NO: 6 is a 5-amino acid-deficient HGF protein (dHGF) from which 5 amino acid residues, that is, the 131st to 135th amino acid residues of the amino acid sequence represented by SEQ ID NO: 5 have been deleted. Both the proteins each having an amino acid sequence represented by SEQ ID NO: 5 or SEQ ID NO: 6 are HGF proteins (natural HGF proteins) naturally present in a human body, and each have, for example, mitogen activity or motogen activity as a HGF protein.

The HGF protein to be used in the present invention includes a protein having a sequence identity of at least about 80% or more with each of the amino acid sequences of HGF proteins (natural HGF proteins) derived from various animals, preferably a protein having a sequence identity of about 90% or more therewith, more preferably a protein having a sequence identity of about 95% or more therewith, the protein having bioactivities (mitogen activity and motogen activity) as a HGF protein. With regard to the amino acid sequences, the term "sequence identity" means consistency in sequence-forming amino acid residue between the sequences of proteins when the primary structures of the proteins are compared to each other, and the term "% or more" means the degree of the consistency.

The fact that the HGF protein has the mitogen activity and the motogen activity described above may be recognized in accordance with, for example, a method described in J. Biol. Chem. 273, 22913-22920, 1998. A protein whose mitogen activity and motogen activity measured in accordance with J. Biol. Chem. 273, 22913-22920, 1998 described above are each typically about 50% or more, preferably about 70% or more, more preferably about 80% or more, still more preferably about 90% or more of that of each of the natural HGF proteins is preferably used.

A protein having the above-mentioned sequence identity with each of the natural HGF proteins is, for example, a protein including an amino acid sequence, which is obtained by substituting, deleting, and/or inserting one to several amino acid residues of, from, and/or into an amino acid sequence represented by SEQ ID NO: 5 or 6, or an amino acid sequence, which is obtained by modifying one to several amino acid residues thereof, the protein having bioactivity as a HGF protein.

The term "several" typically means 1 to 8 (1, 2, 3, 4, 5, 6, 7, or 8), and means typically 8, preferably 6, more preferably 5, still more preferably 3, particularly preferably 2. An amino acid to be inserted or an amino acid to be substituted is preferably a natural amino acid, but may be a non-natural amino acid except 20 kinds of amino acids to be encoded by genes. Although the non-natural amino acid may be any compound as long as the compound has an amino group and a carboxyl group, the acid is, for example, γ-aminobutyric acid.

The substitution of an amino acid residue means the substitution of an amino acid residue in polypeptide with another amino acid residue, and is preferably conservative substitution. The term "conservative substitution" means the substitution of one to several amino acid residues in the polypeptide with other chemically similar amino acid residues so that the activity of the polypeptide may remain substantially unchanged. Such case is, for example, a case in which a hydrophobic amino acid residue is substituted with another hydrophobic amino acid residue, or a case in which a polar amino acid residue is substituted with another polar amino acid residue having the same charge. A functionally similar amino acid that can perform such substitution is known in the art for each amino acid. Examples of the amino acid include: amino acids each having a non-polar (hydrophobic) side chain, such as glycine, alanine, valine, isoleucine, leucine, proline, tryptophan, phenylalanine, and methionine; neutral amino acids each having a polar side chain, such as serine, threonine, tyrosine, glutamine, asparagine, and cysteine; (basic) amino acids each having a positive charge, such as arginine, histidine, and lysine; and (acidic) amino acids each having a negative charge, such as aspartic acid and glutamic acid.

The agents of the present invention may each include only one kind of HGF protein, or may each include two or more kinds of the above-mentioned HGF proteins.

Proteins prepared by various methods may each be used as the HGF protein to be used in each of the agents of the present invention as long as the proteins are each purified to such an extent as to be usable as a medicine. Various methods have been known as methods of preparing the HGF protein, and the protein may be obtained by, for example, extracting a protein from an organ, such as a liver, a spleen, a lung, bone marrow, a brain, a kidney, or a placenta, of a mammalian animal, such as a rat, a cow, a horse, or a sheep, or a hemocyte, such as a thrombocyte or a leukocyte, plasma, or serum of the animal, and purifying the extract.

To extract the HGF protein from the above-mentioned biotissue or the like, and to purify the protein, for example, the following method may be adopted: carbon tetrachloride is intraperitoneally administered to a rat to bring the rat into a hepatitis state; the liver of the rat is removed and pulverized; and the resultant protein is purified by a typical protein purification method, such as column chromatography or HPLC with S-sepharose or heparin sepharose.

In addition, the HGF protein may be obtained by: culturing a primary culture cell or an established cell that produces the HGF protein; and separating the protein from the culture (e.g., a culture supernatant or a culture cell), followed by its purification. Alternatively, a target recombinant HGF protein may be obtained as follows: a gene encoding the HGF protein (preferably DNA formed of a base sequence represented by SEQ ID NO: 1 or 2) is incorporated into an appropriate vector by a genetic engineering approach; the resultant is inserted into a proper host to be subjected to transformation and the protein is obtained from the culture of the transformant (see, for example, Biochem. Biophys. Res. Commun. 180: 1151-1158, 1991; J. Clin. Invest. 87: 1853-1857, 1991; or Protein Expr. Purif. 70: 231-235, 2010).

The above-mentioned host cells is not particularly limited, and various host cells that have heretofore been used in a genetic engineering approach, such as *Escherichia coli, Bacillus subtilis,* yeast, filamentous fungi, and a plant or animal cell, may each be used. For example, when an animal cell is used as the host cells, the HGF protein may be obtained by: subjecting the animal cells, such as a Chinese hamster ovary (CHO) cell, a mouse C127 cell, or a monkey COS cell, to transformation with an expression vector having incorporated thereinto cDNA encoding the amino acid sequence of a human HGF protein; separating the culture supernatant of the transformant; and purifying the supernatant through the above-mentioned column chromatography or the like.

The HGF protein thus obtained may have an amino acid sequence, which is obtained by substituting, deleting, and/or inserting one or a plurality of [e.g., one to several (the term "several" has the same meaning as that described above, and means, for example, 1 to 8, preferably 1 to 6, more preferably 1 to 5, still more preferably 1 to 3, particularly preferably 1 or 2; the same holds true for the following)] amino acids of, from, and/or into the amino acid sequence of a natural HGF protein, as long as the protein has bioactivity as a HGF protein. The substitution is preferably conservative substitution. In addition, similarly, a sugar chain may be substituted, deleted, or inserted into, from, or into the HGF protein. Herein, with regard to the amino acid sequence, the phrase "deleting, substituting, and/or inserting one or a plurality of amino acids" means that such a number of (typically one to several) amino acids that the acids may be produced by a well-known technical method, such as a genetic engineering approach or a site-directed mutagenesis method, or naturally occur are, for example, deleted, substituted, and/or inserted. The HGF protein into, from, or into which the sugar chain has been substituted, deleted, or inserted refers to, for example, a HGF protein obtained by treating the sugar chain added to the natural HGF protein with an enzyme or the like to delete the sugar chain, a protein obtained by subjecting the amino acid sequence of a site to which the sugar chain is to be added in the natural HGF protein to mutation so that the sugar chain may not be added thereto, or a protein obtained by subjecting the amino acid sequence of the natural HGF protein to mutation so that the sugar chain may be added to a site different from its natural site to which the sugar chain is to be added.

When the HGF protein to be used in the present invention is applied to a human, the above-mentioned human-derived protein is suitably used. However, a HGF protein derived from a mammalian animal except a human (e.g., a monkey, a cow, a horse, a pig, a sheep, a dog, a cat, a rat, a mouse, a rabbit, a hamster, a guinea pig, or a chimpanzee) may be used. Examples of such HGF protein include, but not limited to, a HGF protein derived from a mouse (e.g., Accession No. AAB31855, NP_034557, BAA01065, or BAA01064), a HGF protein derived from a rat [e.g., Accession No. NP_58713], a HGF protein derived from a cow (e.g., Accession No. NP_001026921, XP874086, or BAD02475), a HGF protein derived from a cat (e.g., Accession No. NP_001009830, BAC10545, or BAB21499), a HGF protein derived from a dog (e.g., Accession No. NP_001002964 or BAC57560), and a HGF protein derived from a chimpanzee (e.g., Accession No. XP519174) each registered in the NCBI database or the like.

The C-terminal of the HGF protein to be used in the present invention may be any one of a carboxyl group (-COOH), a carboxylate [-COOM (M represents a metal)], an amide (-CONH₂), or an ester (-COOR). Herein, as the R in the ester, there may be used, for example: a C1-6 alkyl group, such as a methyl, ethyl, n-propyl, isopropyl, or n-butyl group; a C3-8 cycloalkyl group, such as a cyclopentyl or cyclohexyl group; a C6-12 aryl group, such as a **phenyl or α**-naphthyl group; a C7-14 aralkyl group, such as a phenyl-C1-2 alkyl group, such as a benzyl or phenethyl group, or **an α**-naphthyl-C1-2 alkyl group such as **an α**-naphthylmethyl group; or a C2-6 alkanoylmethyl group, such as an acetyloxymethyl or pivaloyloxymethyl group. In the case where the HGF protein to be used in the present invention has a carboxyl group or a carboxylate at a site except the C-terminal, a protein obtained by amidating or esterifying the carboxyl group or the carboxylate is also included in the HGF protein in the present invention. For example, the ester at the C-terminal described above is used as an ester in this case. Further, the HGF protein to be used in the present invention includes: a protein obtained by protecting the amino group of a methionine residue at an N-terminal in the above-mentioned protein with a protective group (e.g., a C1-6 acyl group, such as a formyl group or a C2-6 alkanoyl group such as an acetyl group); a protein obtained by turning a glutamyl group, which has been produced by the cleavage of the N-terminal side of the above-mentioned protein in a living organism, into pyroglutamic acid; a protein obtained by protecting a reactive group (e.g., -OH, -SH, an amino group, an imidazolyl group, an indolyl group, or a guanidino group) on a side chain of an amino acid in a molecule of the above-mentioned protein with a proper protective group (e.g., a C1-6 acyl group, such as a formyl group or a C2-6 alkanoyl group such as an acetyl group); or a conjugated protein such as a so-called glycoprotein having bonded thereto a sugar chain.

The term "gene encoding the HGF protein" as used in the present invention refers to a gene that can express the above-mentioned HGF protein. DNA holding the gene encoding the HGF protein (hereinafter sometimes referred to as "DNA encoding the HGF protein") is described in, for example, Nature, 342, 440 (1989); JP 2777678 B2; Biochem. Biophys. Res. Commun., 1989, vol. 163, pp. 967-973; or Proc. Natl. Acad. Sci. U.S.A., 1991, vol. 88 (No. 16), pp. 7001-7005, and is preferably, for example, DNA encoding a human-derived HGF protein registered as Accession No. M69718, M73240, AC004960, AY246560, M29145, or M73240 in GenBank, EMBL, or DDBJ.

In addition, when the DNA encoding the HGF protein to be used in the present invention is applied to a human, the above-mentioned human-derived protein is suitably used. However, DNA encoding a HGF protein derived from a mammalian animal except a human (e.g., a monkey, a cow, a horse, a pig, a sheep, a dog, a cat, a rat, a mouse, a rabbit, a hamster, a guinea pig, or a chimpanzee) may be used.

Examples of such DNA encoding the HGF protein include, but not limited to, DNA encoding a HGF protein derived from a mouse (e.g., Accession No. S71816, NM_010427, D10213, or D10212), DNA encoding a HGF protein derived from a rat (e.g., Accession No. NM_017017), DNA encoding a HGF protein derived from a cow (e.g., Accession No. NM_001031751 or AB110822), DNA encoding a HGF protein derived from a cat (e.g., Accession No. NM_001009830, AB080187, or AB046610), DNA encoding a HGF protein derived from a dog (e.g., Accession No. NM_001002964 or AB090353), and DNA encoding a HGF protein derived from a chimpanzee (e.g., Accession No. XM_519174) each registered in the NCBI database or the like.

Further, a preferred specific example of the DNA encoding the HGF protein is DNA having a base sequence represented by SEQ ID NO: 1 or 2. Herein, the base sequence represented by SEQ ID NO: 1 corresponds to a base sequence positioned at the 73rd to 2,259th positions of the base sequence of Accession No. M60718, and DNA formed of the base sequence corresponds to DNA encoding a HGF protein formed of an amino acid sequence represented by SEQ ID NO: 3. In addition, in a DNA recombination technology, when the HGF protein (SEQ ID NO: 3) expressed and produced in a cell is secreted to the outside of the cells, its signal sequence is cleaved to provide a HGF protein formed of an amino acid sequence represented by SEQ ID NO: 5. Accordingly, the DNA formed of the base sequence represented by SEQ ID NO: 1 corresponds to DNA encoding (producing) the HGF protein formed of the amino acid sequence represented by SEQ ID NO: 5. The base sequence represented by SEQ ID NO: 2 corresponds to a base sequence positioned at the 66th to 2,237th positions of the base sequence of Accession No. M73240, and DNA formed of the base sequence corresponds to DNA encoding a HGF protein formed of an amino acid sequence represented by SEQ ID NO: 4. For such HGF protein (SEQ ID NO: 4) as well, in the DNA recombination technology, when the protein is secreted to the outside of the cells, its signal sequence is cleaved to provide a HGF protein formed of an amino acid sequence represented by SEQ ID NO: 6. Accordingly, the DNA formed of the base sequence represented by SEQ ID NO: 2 corresponds to DNA encoding (producing) the HGF protein formed of the amino acid sequence represented by SEQ ID NO: 6.

### (Substance having c-Met Phosphorylation Action comparable to that of HGF Protein and Gene encoding the Substance)

It has been known that a HGF protein, which is a substance having a c-Met phosphorylation action, induces the phosphorylation of a c-Met receptor to provide an anti-apoptosis action, an angiogenesis action, a morphogenesis action, a cell division-promoting action, an axon-elongating action, or the like (see Proc Jpn Acad Ser B Phys Biol Sci. 2010; 86(6): 588-610).

That is, a substance having a c-Met phosphorylation action comparable to that of the HGF protein may be used as an effective component for each of the agents of the present invention as in the HGF protein.

Examples of the substance having a c-Met phosphorylation action comparable to that of the HGF protein may include, but not particularly limited to, the following substances. Preferred examples thereof include: low-molecular-weight compounds, such as ANG-3777 (Angion Biomedica Corp.: a Hepatocyte Growth Factor (HGF) Mimetic) and ATH-1017 (Athira Pharma, Inc.); cyclic peptide dimers, such as aML5, aMD4, and aMD5; a HGF alternative peptide (product code: PG-001; a c-Met agonist; PeptiGrowth Inc.); an anti-Met antibody AGMB-101 (AgomAb Therapeutics: AGMB-101 is a full MET agonist); HGF molecular fragment variants, such as NK1 dimer and K1K1; a DNA aptamer and an RNA aptamer; Internalin (Listeria protein); and a peptide having activity substantially identical in quality to that of the HGF protein.

As the peptide having activity substantially identical in quality to that of the HGF protein (hereinafter sometimes abbreviated as "HGF partial peptide"), any partial peptide of the above-mentioned HGF protein, the partial peptide having activity substantially identical in quality to that of the HGF protein, may be used. In the present invention, with regard to the number of the amino acids of the HGF partial peptide, for example, a peptide containing at least about 20 or more, preferably about 50 or more, more preferably about 100 or more amino acid sequences out of the amino acid sequences for forming the above-mentioned HGF protein is preferred. Specifically, for example, a peptide represented by a sequence ranging from the N-terminal hairpin loop of the HGF of a human HGF amino acid sequence to the first Kringle domain thereof is preferred.

In the HGF partial peptide of the present invention, its C-terminal may be any one of a carboxyl group (-COOH), a carboxylate [-COOM], an amide (-CONH₂), or an ester (-COOR). Further, as in the above-mentioned HGF protein, the HGF partial peptide includes: a partial peptide obtained by protecting the amino group of a methionine residue at the N-terminal of the above-mentioned partial peptide with a protective group; a partial peptide obtained by turning Gln, which has been produced by the cleavage of the N-terminal side thereof in a living organism, into pyroglutamic acid; a partial peptide obtained by protecting a substituent on a side chain of an amino acid in a molecule thereof with a proper protective group; or a conjugated peptide such as a so-called glycopeptide having bonded thereto a sugar chain.

A gene encoding the substance having a c-Met phosphorylation action to be used in the present invention is, for example, a gene encoding the above-mentioned peptide having a c-Met phosphorylation action.

### (Sustained Release Carrier)

A sustained release carrier of the present invention is not particularly limited as long as the carrier can sustained-release a HGF protein or a substance having a c-Met phosphorylation action comparable to that of the HGF protein, or a gene encoding the hepatocyte growth factor protein or a gene encoding the substance having a c-Met phosphorylation action comparable to that of the HGF protein (hereinafter sometimes abbreviated as "effective component of the present invention" in this description) in a lesion site of a spinal cord.

The shape of the sustained release carrier may be, for example, a membrane shape (a sheet shape or a film shape), a sponge shape, a gel shape, a spherical shape, a particle shape, or a fibrous shape, but is not particularly limited thereto.

The sustained release carrier may be produced in accordance with a known method. A biodegradable polymer to be used in the sustained release carrier may be appropriately selected from known biodegradable polymers.

Examples thereof may include: polysaccharides, such as starch, dextran, hyaluronan (hyaluronic acid) or a salt thereof, and chitosan; proteins, such as atelocollagen, collagen, and gelatin; polyamino acids, such as polyglutamic acid, polylysine, polyleucine, polyalanine, and polymethionine; polyesters, such as polylactic acid, polyglycolic acid, a lactic acid-glycolic acid copolymer, polycaprolactone, poly-**β**-hydroxybutyric acid, polymalic acid, polyacid anhydride, and a fumaric acid-polyethylene glycol-vinylpyrrolidone copolymer; polyorthoesters; polyalkyl cyanoacrylic acids such as polymethyl-**α**-cyanoacrylic acid; and polycarbonates, such as polyethylene carbonate or polypropylene carbonate.

A preferred sustained release carrier is a lactic acid-glycolic acid copolymer or collagen.

In the case where the lactic acid-glycolic acid copolymer is used, its composition ratio (lactic acid/glycolic acid) (mol%) varies depending on its sustained release period, but for example, when the sustained release period is from about 2 weeks to about 3 months, preferably from about 2 weeks to about 1 month, the ratio is from about 100/0 to about 50/50. The weight-average molecular weight of the lactic acid-glycolic acid copolymer is generally from about 5,000 to about 20,000. The lactic acid-glycolic acid copolymer may be produced in accordance with a known production method such as a production method described in JP 61-28521 A. Although a blending ratio between the biodegradable polymer and the HGF protein is not particularly limited, the ratio of the HGF protein to the biodegradable polymer is, for example, from about 0.01 w/w% to about 30 w/w%.

As the lactic acid-glycolic acid copolymer, there may be preferably used, for example, Neuro-Spinal Scaffold (highly porous bioresorbable polymer composed of poly(lactic-co-glycolic acid)-b-poly-(I-lysine); InVivo Therapeutics).

In this description, the term "%" means "mass%" unless otherwise stated.

When the collagen is used, atelocollagen is preferred. For example, a collagen sponge sheet or an artificial dermis (PELNAC; Gunze Limited) having a sponge-like structure may be preferably used as the atelocollagen. For example, gelatin (preferably negatively charged gelatin) may be incorporated into the atelocollagen. A negatively charged gelatin-containing collagen sponge sheet is, for example, PELNAC G PLUS (Gunze Limited) obtained by incorporating 10% of the negatively charged gelatin into the PELNAC. When the PELNAC G PLUS is used, a positively charged substance such as a HGF protein can be easily bonded to the negatively charged gelatin incorporated thereinto, and hence the HGF protein can be sustained-released along with the decomposition of the gelatin.

The sustained release carrier can carry the effective component of the present invention through, for example, the following carrying method: a solution containing the effective component of the present invention is impregnated into the sustained release carrier; or the solution is applied to, spread on, or injected into the sustained release carrier. With regard to the carrying amount of the effective component, the carrier preferably contains at least the dose of the effective component of the present invention.

In addition, the gene encoding the HGF protein or the gene encoding the substance having a c-Met phosphorylation action comparable to that of the HGF protein is carried by the sustained release carrier in the form of a recombinant expression vector having incorporated thereinto DNA holding the gene. Examples of such expression vector include DNA viruses or RNA viruses, such as a naked plasmid, a detoxified retrovirus, an adenovirus, an adeno-associated virus, a herpesvirus (e.g., type I herpes simplex virus), a vaccinia virus, a poxvirus, a poliovirus, a Sindbis virus, a Sendai virus, SV40, and an immunodeficiency virus (HIV). Of those, for example, a naked plasmid, a type I herpes simplex virus (HSV-1) vector, a Sendai virus-envelope (HVJ-E) vector, an adenovirus vector, or an adeno-associated virus (AAV) vector is preferred.

### (Pluripotent Stem Cell)

Although the pluripotent stem cells of the present invention is not particularly limited as long as the cells can exhibit the effects of the agents of the present invention, examples of the pluripotent stem cells include: a differentiated cell-derived pluripotent stem cells (see JP 2009-215191 A) obtained by forcibly expressing each of an Oct3/4 gene, a Sox2 gene, and a Klf4 gene with a differentiated cell; a pluripotent stem cell (see JP 5603282 B2); an embryonic stem cell (ES cell); and an induced pluripotent stem cell (iPS cell).

A neural stem cell means a cell having a self-renewal ability and an ability to differentiate into a neural progenitor cell. In addition, the neural progenitor cells refer to a cell, which is undifferentiated but is differentiated from the neural stem cells by one stage, and which performs the self-reproduction of itself to finally differentiate into a nerve cell. The neural stem cell or the neural progenitor cells are preferably obtained by the induction of differentiation from the pluripotent stem cells.

A method of inducing the differentiation of the pluripotent stem cells into the neural stem cells or the neural progenitor cells are not particularly limited, and a method to be typically performed may be adopted. For example, a neurosphere may be obtained by subjecting the pluripotent stem cells to suspension culture in a culture medium containing at least one kind of a bFGF or an EGF. In addition, the following may be repeated a plurality of times: the resultant neurosphere is dissociated into single cells; and the cells are subjected to suspension culture in a culture medium containing the bFGF again to form a neurosphere again.

For example, a human induced pluripotent cells line, such as a 201B7 cell, a 201B7-Ff cell, a 253G1 cell, a 253G4 cell, a 1201C1 cell, a 1205D1 cell, a 1210B2 cell, or a 1231A3 cell, is available (see WO 2021/045217 A1).

An iPS cell-derived neural stem and/or progenitor cells may be preferably, for example, a neural stem cell and/or a neural progenitor cell derived from a human umbilical cord blood-derived human iPS cells (YZWJs513 hiPSC).

### (Method of implanting Sustained Release Carrier)

A method of implanting the sustained release carrier is not particularly limited. In addition, the method is described by taking the HGF protein as an example, but a sustained release carrier containing any other effective component may be similarly implanted.

The sustained release carrier carrying the HGF protein is preferably implanted in a lesion site of a spinal cord (in particular, a cavity site at which the spinal cord has been transected). In addition, although the dose of the HGF protein is appropriately selected in accordance with, for example, the degree of a disease or an age, the amount of the HGF protein is typically from 1 µg to 100 mg, preferably from 10 µg to 50 mg per administration. In addition, the following administration methods may each be adopted: the entire amount of the protein is administered once; or the protein is administered a plurality of times at intervals.

An administration timing is also appropriately selected in accordance with, for example, the degree of a disease or an age, and the HGF protein may be administered, for example, immediately after the traumatism of the spinal cord or at any time thereafter.

However, as described in Examples below, the agents of the present invention each have a therapeutic effect on chronic incomplete spinal cord injury or chronic complete spinal cord injury, and hence the agents can each be administered not only in an acute phase lasting for about 2 weeks from immediately after the traumatism and a subacute phase 2 to 3 weeks after the traumatism but also in a chronic phase after the subacute phase.

### (Method of administering Pluripotent Stem Cell)

A method of administering the pluripotent stem cells includes grafting or injection, but is not particularly limited thereto. Examples thereof may include: a method including administering the cells to the sustained release carrier that has already been implanted described above (in particular, a method including administering the pluripotent stem cells to a plurality of sites of the carrier); and a method including administering the pluripotent stem cells to a lesion site of a spinal cord (in particular, a cavity site at which the spinal cord has been transected) before or after the implantation of the sustained release carrier. In addition, the cells may be administered by intravenous injection or the like to be caused to reach the lesion site of the spinal cord.

Although the pluripotent stem cells may be administered at a timing before the implantation of the sustained release carrier, the cells are preferably administered after the implantation of the sustained release carrier. Examples of the timing at, or the time period for, which the HGF protein is sustained-released from the sustained release carrier after the implantation of the sustained release carrier can be given.

The sustained release is started typically 1 day to 40 days (1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 days), preferably 1 day to 30 days after the implantation of the sustained release carrier.

In addition, when the HGF protein is administered a plurality of times, the timing at which the pluripotent stem cells are administered may be added to the above-mentioned values.

The number of times of the administration of the pluripotent stem cells are not particularly limited, and is at least one or more, typically from one to about five. When the HGF protein is administered a plurality of times, the number of times of the administration of the pluripotent stem cells may be appropriately increased.

Although the number of the pluripotent stem cells to be administered is not particularly limited, the number is at least 100,000 cells/per administration or more (typically from 100,000 cells/per administration to 50,000,000 cells/per administration, from 100,000 cells/per administration to 10,000,000 cells/per administration, from 100,000 cells/per administration to 5,000,000 cells/per administration, from 200,000 cells/per administration to 3,000,000 cells/per administration, or from 300,000 cells/per administration to 2,500,000 cells/per administration).

### (Therapeutic Agent for Spinal Cord Injury)

The therapeutic agent for spinal cord injury of the present invention includes the following:
(1) (a) a HGF protein or a substance having a c-Met phosphorylation action comparable to that of the HGF protein, or (b) a gene encoding the HGF protein or a gene encoding the substance having a c-Met phosphorylation action comparable to that of the HGF protein;
(2) a carrier configured to carry, and to be capable of sustained-releasing, the (a) or the (b); and
(3) a pluripotent stem cell.

In addition, another aspect of the therapeutic agent for spinal cord injury of the present invention includes the following:
(1) a HGF protein, and a carrier configured to carry, and to be capable of sustained-releasing, the HGF protein.

The carrier carrying the HGF protein is implanted in a lesion site of a spinal cord, and then an iPS cell-derived neural stem and/or progenitor cells are administered to the carrier or the lesion site of the spinal cord.

In addition, another aspect of the therapeutic agent for spinal cord injury of the present invention includes the following:
(1) an iPS cell-derived neural stem and/or progenitor cells.

The iPS cell-derived neural stem and/or progenitor cells are administered to the lesion site of the spinal cord in which the carrier carrying the HGF protein has been implanted.

### (Therapeutic Method for Spinal Cord Injury)

A therapeutic method for spinal cord injury of the present invention includes the following steps:
(1) a step of implanting, in a lesion site of a spinal cord of a target having spinal cord injury, a carrier configured to carry, and to be capable of sustained-releasing,
   (a) a HGF protein or a substance having a c-Met phosphorylation action comparable to that of the HGF protein, or
   (b) a gene encoding the HGF protein or a gene encoding the substance having a c-Met phosphorylation action comparable to that of the HGF protein; and
(2) a step of administering a pluripotent stem cell to the carrier after the implantation in the step (1) or the lesion site of the spinal cord.

### (Kit for treating Spinal Cord Injury)

A kit for treating spinal cord injury of the present invention includes the following:
(1) (a) a HGF protein or a substance having a c-Met phosphorylation action comparable to that of the HGF protein, or (b) a gene encoding the HGF protein or a gene encoding the substance having a c-Met phosphorylation action comparable to that of the HGF protein;
(2) a carrier configured to carry, and to be capable of sustained-releasing, the (a) or the (b); and
(3) a pluripotent stem cell,

The carrier configured to carry the (a) or the (b) is implanted in a lesion site of a spinal cord of a spinal cord injury patient, and the pluripotent stem cells are administered to the carrier after the implantation or to the lesion site of the spinal cord.

As a preferred aspect of each of the therapeutic agent for spinal cord injury, therapeutic method for spinal cord injury, and kit for treating spinal cord injury of the present invention, the substance having a c-Met phosphorylation action is a HGF protein, the pluripotent stem cells are an iPS cell-derived neural stem and/or progenitor cells, and the spinal cord injury is chronic incomplete spinal cord injury or chronic complete spinal cord injury.

Further, as a preferred aspect of each of the therapeutic agent for spinal cord injury, therapeutic method for spinal cord injury, and kit for treating spinal cord injury of the present invention, the carrier configured to carry, and to be capable of sustained-releasing, the HGF protein is implanted in a lesion site, and the iPS cell-derived neural stem and/or progenitor cells are administered to the carrier after the implantation or to the lesion site.

As an example of treatment through use of each of the therapeutic agent for spinal cord injury, therapeutic method for spinal cord injury, and kit for treating spinal cord injury of the present invention, the regeneration of an injured spinal cord is promoted and/or an accessory symptom accompanying the spinal cord injury is alleviated.

The promotion of the regeneration of the injured spinal cord is, for example, the promotion of axon extension, an increase in number of nerve fibers, the neurogenesis of an endogenous stem cells, angiogenesis, the inhibition of scar formation, the inhibition of inflammation, angiogenesis, and/or the inhibition of spinal cord cavitation.

The accessory symptom accompanying the injured spinal cord is, for example, motor dysfunction and/or voiding dysfunction.

The motor dysfunction is due to, for example, the motor dysfunction of a lower limb or a reduction in muscle mass thereof.

### (Agent for Alleviating Voiding dysfunction in Chronic Incomplete Spinal Cord Injury or Chronic Complete Spinal Cord Injury)

An agent for alleviating voiding dysfunction in chronic incomplete spinal cord injury or chronic complete spinal cord injury of the present invention includes the following:
(1) a HGF protein, and a carrier configured to carry, and to be capable of sustained-releasing, the HGF protein; and
(2) an iPS cell-derived neural stem and/or progenitor cells.

The carrier carrying the HGF protein is implanted in a lesion site of a spinal cord, and the iPS cell-derived neural stem and/or progenitor cells are administered to the carrier after the implantation or to the lesion site of the spinal cord.

### (Agent for Alleviating Motor Dysfunction in Chronic Incomplete Spinal Cord Injury or Chronic Complete Spinal Cord Injury)

An agent for alleviating motor dysfunction in chronic incomplete spinal cord injury or chronic complete spinal cord injury of the present invention includes the following:
(1) a HGF protein, and a carrier configured to carry, and to be capable of sustained-releasing, the HGF protein; and
(2) an iPS cell-derived neural stem and/or progenitor cells.

The carrier carrying the HGF protein is implanted in a lesion site of a spinal cord, and the iPS cell-derived neural stem and/or progenitor cells are administered to the carrier after the implantation or to the lesion site of the spinal cord.

The motor dysfunction is, for example, motor dysfunction of a lower limb.

### (Lower Limb Muscle Mass-increasing Agent in Chronic Incomplete Spinal Cord Injury or Chronic Complete Spinal Cord Injury)

A lower limb muscle mass-increasing agent in chronic incomplete spinal cord injury or chronic complete spinal cord injury of the present invention includes the following:
(1) a HGF protein, and a carrier configured to carry, and to be capable of sustained-releasing, the HGF protein; and
(2) an iPS cell-derived neural stem and/or progenitor cells.

The carrier carrying the HGF protein is implanted in a lesion site of a spinal cord, and the iPS cell-derived neural stem and/or progenitor cells are administered to the carrier after the implantation or to the lesion site of the spinal cord.

### (Spinal Cord Cavitation Inhibitor in Chronic Incomplete Spinal Cord Injury or Chronic Complete Spinal Cord Injury)

A spinal cord cavitation inhibitor in chronic incomplete spinal cord injury or chronic complete spinal cord injury of the present invention includes the following:
(1) a HGF protein, and a carrier configured to carry, and to be capable of sustained-releasing, the HGF protein; and
(2) an iPS cell-derived neural stem and/or progenitor cells.

The carrier carrying the HGF protein is implanted in a lesion site of a spinal cord, and the iPS cell-derived neural stem and/or progenitor cells are administered to the carrier after the implantation or to the lesion site of the spinal cord.

### (Anti-inflammatory Agent for Lesion Site in Chronic Incomplete Spinal Cord Injury or Chronic Complete Spinal Cord Injury)

An anti-inflammatory agent for a lesion site in chronic incomplete spinal cord injury or chronic complete spinal cord injury of the present invention includes the following:
(1) a HGF protein, and a carrier configured to carry, and to be capable of sustained-releasing, the HGF protein; and
(2) an iPS cell-derived neural stem and/or progenitor cells.

The carrier carrying the HGF protein is implanted in a lesion site of a spinal cord, and the iPS cell-derived neural stem and/or progenitor cells are administered to the carrier after the implantation or to the lesion site of the spinal cord.

### (Angiogenesis Promoter for Lesion Site in Chronic Incomplete Spinal Cord Injury or Chronic Complete Spinal Cord Injury)

An angiogenesis promoter for a lesion site in chronic incomplete spinal cord injury or chronic complete spinal cord injury of the present invention includes the following:
(1) a HGF protein, and a carrier configured to carry, and to be capable of sustained-releasing, the HGF protein; and
(2) an iPS cell-derived neural stem and/or progenitor cells.

The carrier carrying the HGF protein is implanted in a lesion site of a spinal cord, and the iPS cell-derived neural stem and/or progenitor cells are administered to the carrier after the implantation or to the lesion site of the spinal cord.

### (Scar Formation Inhibitor for Lesion Site in Chronic Incomplete Spinal Cord Injury or Chronic Complete Spinal Cord Injury)

A scar formation inhibitor for a lesion site in chronic incomplete spinal cord injury or chronic complete spinal cord injury of the present invention includes the following:
(1) a HGF protein, and a carrier configured to carry, and to be capable of sustained-releasing, the HGF protein; and
(2) an iPS cell-derived neural stem and/or progenitor cells.

The carrier carrying the HGF protein is implanted in a lesion site of a spinal cord, and the iPS cell-derived neural stem and/or progenitor cells are administered to the carrier after the implantation or to the lesion site of the spinal cord.

### (Axon Extension Promoter for Lesion Site in Chronic Incomplete Spinal Cord Injury or Chronic Complete Spinal Cord Injury)

An axon extension promoter for a lesion site in chronic incomplete spinal cord injury or chronic complete spinal cord injury of the present invention includes the following:
(1) a HGF protein, and a carrier configured to carry, and to be capable of sustained-releasing, the HGF protein; and
(2) an iPS cell-derived neural stem and/or progenitor cells.

The carrier carrying the HGF protein is implanted in a lesion site of a spinal cord, and the iPS cell-derived neural stem and/or progenitor cells are administered to the carrier after the implantation or to the lesion site of the spinal cord.

### Examples

The present invention is described in detail below by way of specific examples. However, the present invention is not limited to these Examples.

All animal experiments were performed in accordance with the guideline of The Keio University Institutional Animal Care and Use Committee (approval number of 7,207 of Keio University).

### Example 1

In this Example, the effect of a hepatocyte growth factor (HGF) protein on an injured spinal cord in spinal cord regeneration, and the effect of a scaffold having both of a function as a scaffold and the property by which a drug was sustained-released with charge were recognized.

### (Material and Method)

A recombinant human HGF protein used here, which was formed of an amino acid sequence represented by SEQ ID NO: 6, was produced by using a CHO cell in accordance with a method described in Biochem. Biophys. Res. Commun. 180: 1151-1158, 1991.

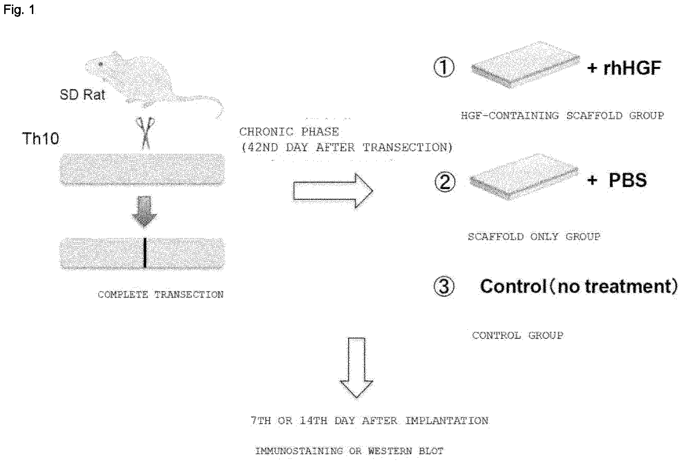

A complete spinal cord injury model was produced by transecting the high spinal cord of the tenth thoracic vertebra of a female Sprague-Dawley rat (having a body weight of from 160 g to 200 g). On a 42nd day (corresponding to a chronic phase) after the injury, a total of three groups were prepared as follows (see FIG. 1, reference: J Surg Res., 2018 (221), 173-182): a HGF protein-containing sustained release-type collagen gelatin scaffold (HGF+scaffold group) was surgically implanted in a cavity formed at a transected site, and as a control group, a collagen gelatin scaffold containing phosphate buffered saline (scaffold group) was implanted in a lesion, or a control group in which no treatment was performed was prepared.

**The recombinant human HGF protein was used at a concentration of 10 mg/10 µL.**

PELNAC G PLUS (a product of Gunze Limited) having a sheet shape measuring 5 mm by 5 mm by 3 mm was used as the collagen gelatin scaffold. The size of the scaffold to be implanted was changed at all times in accordance with the size of the cavity.

The HGF protein was carried on the collagen gelatin scaffold by a method including impregnating the HGF protein into the collagen gelatin scaffold.

The spinal cord around the position at which each of the scaffolds had been implanted was collected on a 7th day and/or a 14th day after the implantation. Then, the measurement of the intensity of phosphorylated c-Met, the evaluation of a newborn blood vessel in a lesion epicenter (RECA-1 positive area), the evaluation of an anti-inflammatory action (Arginase-1, Arginase-1 positive cells/iba1 positive cells, and TNF-a), the evaluation of a neuroprotective action (BDNF), the evaluation of the inhibition of scar formation (TGF-b), and the evaluation of an axon-regenerating or axon-reextending action (co-staining with Tuj-1 and pGAP43) were performed by using immunostaining and a Western blot.

References of the respective evaluation methods were as follows: "Regulation of axon growth by the JIP1– AKT axis, 2014," "Combined Action of GDNF and HGF Up-Regulates Axonal Growth by Increasing ERK1/2 Phosphorylation, 2018," "PI3K-GSK3 signaling regulates mammalian axon regeneration by inducing the expression of Smad1, 2013," and "The discovery of Hepatocyte Growth Factor (HGF) and its significance for cell biology, life sciences and clinical medicine, 2010."

### (Recognition of Sustained-releasing Effect on HGF Protein exhibited by Scaffold)

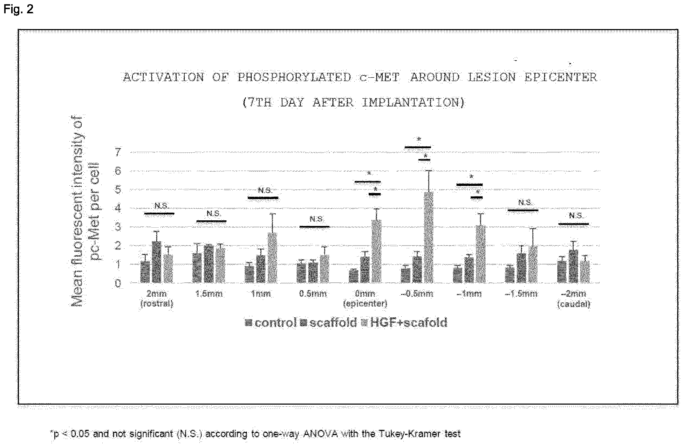

As shown in the graph of FIG. 2, the phosphorylated c-Met was significantly detected in the HGF protein-containing scaffold group at a position caudal to the lesion epicenter by 1 mm. The detection of the phosphorylated c-Met means that an activating action exhibited by the HGF protein lasted, and hence it was recognized that the HGF protein-containing scaffold sustained-released the HGF protein until at least the 7th day after the implantation.

Accordingly, the HGF protein-containing scaffold has a sustained-releasing effect on the HGF protein.

In addition, a scaffold containing a substance having a c-Met phosphorylation action comparable to that of the HGF protein can continuously perform the phosphorylation of c-Met as in the HGF protein-containing scaffold.

### (Evaluation of Angiogenesis-promoting Action in Lesion Epicenter in HGF Protein-containing Scaffold)

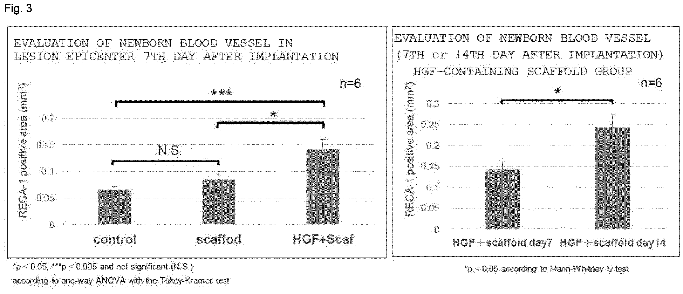

As shown in the left graph of FIG. 3, in the HGF protein-containing scaffold group, newborn blood vessels whose number was significantly larger than those of the control groups were observed. Further, in the HGF protein-containing scaffold group on the 14th day, newborn blood vessels whose number was larger than that on the 7th day were observed (the right of FIG. 3).

Accordingly, the HGF protein-containing scaffold has an angiogenesis-promoting action in the lesion epicenter.

### (Evaluation of Anti-inflammatory Action exhibited by HGF Protein-containing Scaffold)

An anti-inflammatory action exhibited by the HGF protein-containing scaffold was evaluated by the measurement of the concentration of Arginase-1, which was a marker for anti-inflammatory microglia and macrophages each showing a neuroprotective action, the ratio of cells that were Arginase-1 positive to microglia and macrophages that were ionized calcium binding adapter protein 1 (iba1) positive, and the measurement of the concentration of TNF-a, which was an inflammatory cytokine.

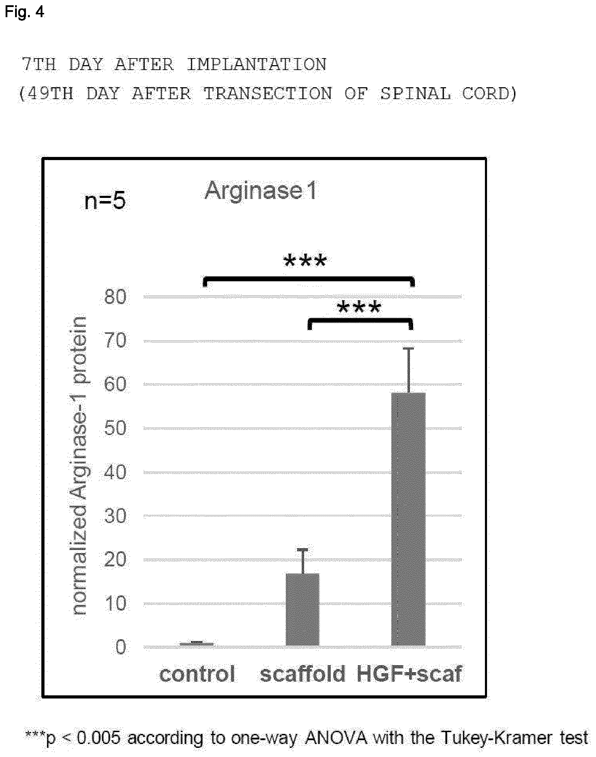

As is apparent from the graph of FIG. 4, it was recognized that the concentration of Arginase-1 in the HGF protein-containing scaffold group increased as compared to those in the control groups.

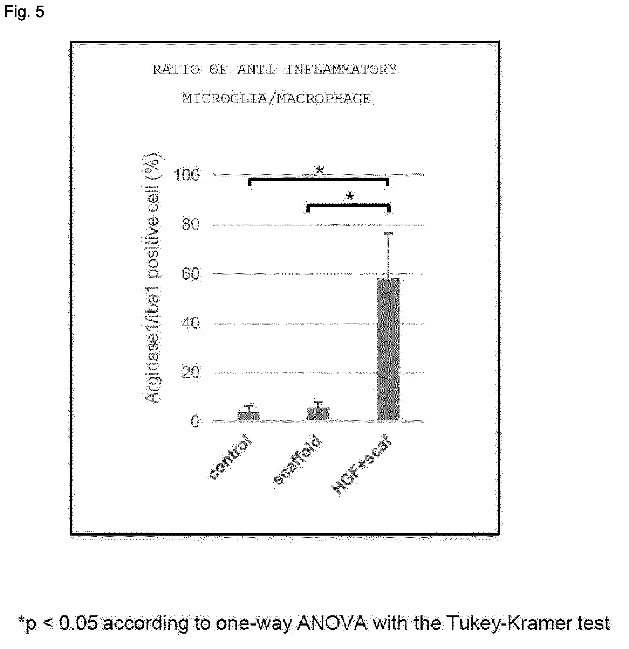

As is apparent from the graph of FIG. 5, it was recognized that the ratio of the cells that were Arginase-1 positive to the iba1 positive cells in the HGF protein-containing scaffold group was significantly higher than those in the control groups.

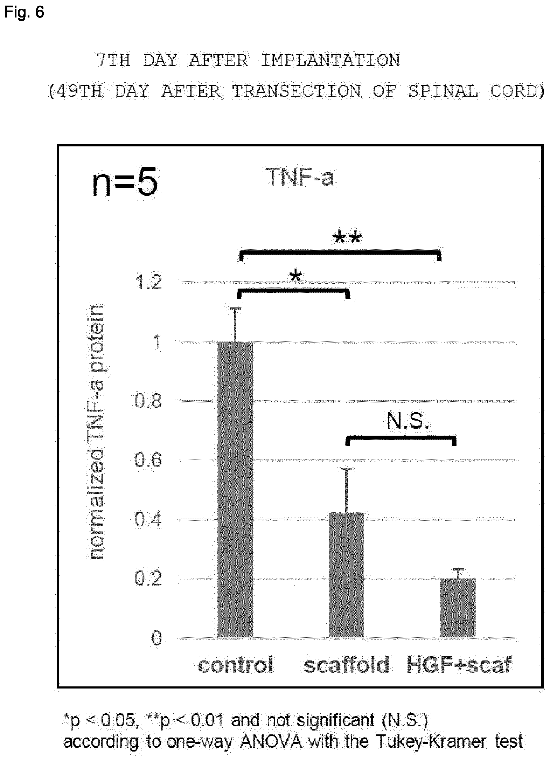

As is apparent from the graph of FIG. 6, it was recognized that the concentration of TNF-a, which was an inflammatory cytokine, in the HGF protein-containing scaffold group was lower than those in the control groups.

Accordingly, the HGF protein-containing scaffold has an anti-inflammatory action and effect through the sustained release of the HGF protein. Further, it was recognized that the HGF protein-containing scaffold was able to increase a grafted cell survival rate by virtue of the TNF-a-inhibiting action of the HGF protein-containing scaffold.

### (Evaluation of Neuroprotective Action exhibited by HGF Protein-containing Scaffold)

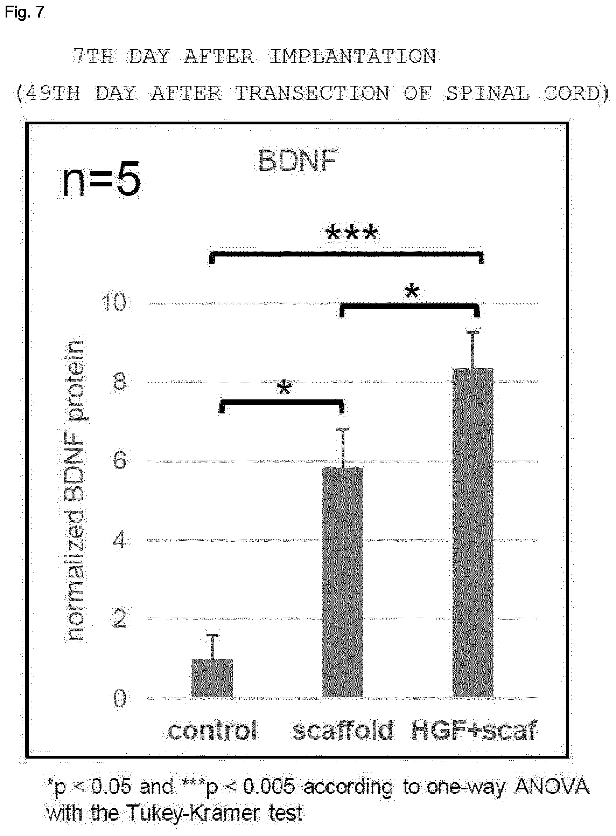

As shown in the graph of FIG. 7, it was recognized that the concentration of BDNF, which was a neurotrophic factor, in the HGF protein-containing scaffold group increased as compared to those in the control groups.

Accordingly, the HGF protein-containing scaffold has a neuroprotective action and effect through the sustained release of the HGF protein.

### (Evaluation of Inhibition of Scar Formation by HGF Protein-containing Scaffold)

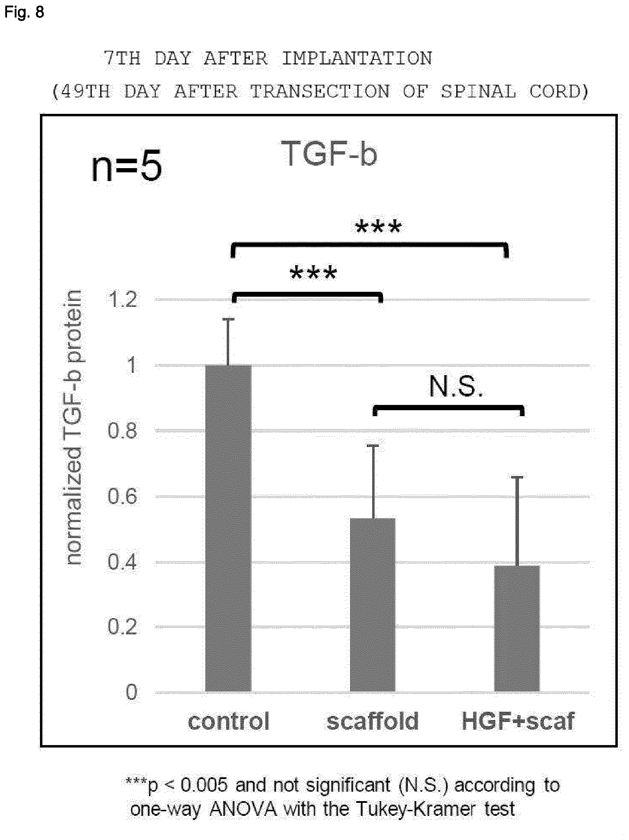

As shown in the graph of FIG. 8, it was recognized that the concentration of TGF-b, which was a factor for forming a glial scar, in the HGF protein-containing scaffold group reduced as compared to those in the control groups.

Accordingly, the HGF protein-containing scaffold has a scar formation-inhibiting (in particular, a glial scar formation-inhibiting) effect through the sustained release of the HGF protein.

### (Evaluation of Axon-regenerating or Axon-reextending Action exhibited by HGF Protein-containing Scaffold)

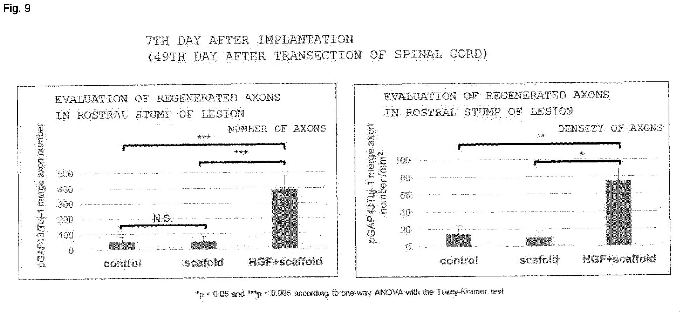

The number of regenerated axons and the density of the regenerated axons in the rostral stump of a lesion in an axial image obtained by the co-staining of the rostral stump of the lesion with Tuj-1 and pGAP43, which is a marker for the regenerated axons, are shown in FIG. 9.

As shown in the graphs of FIG. 9, it was recognized that the number of the regenerated axons (the left of FIG. 9) and the density of the regenerated axons (the right of FIG. 9) in the HGF protein-containing scaffold group were larger than those in the control groups. In addition, comparison between the numbers of the regenerated axons in the HGF protein-containing scaffold group on the 7th day and the 14th day after the implantation recognized that the regenerated axons further increased on the 14th day (not shown).

Accordingly, the HGF protein-containing scaffold has a promoting effect on axon extension through the sustained release of the HGF protein.

### (General Remarks on Example 1)

It was recognized that the HGF protein-containing scaffold had the following effects:
(1) the effect by which the HGF protein was sustained-released for at least 14 days;
(2) an angiogenesis-promoting action in a lesion site of a spinal cord (in particular, an angiogenesis-promoting action in a lesion epicenter);
(3) an anti-inflammatory action in the lesion site of the spinal cord;
(4) an increasing action on the survival rate of grafted cells;
(5) a neuroprotective action in the lesion site of the spinal cord;
(6) scar formation inhibition in the lesion site of the spinal cord; and
(7) a promoting action on axon extension in the lesion site of the spinal cord (in particular, an increasing action on the number of regenerated axons and an increasing action on the density of the regenerated axons).

### Example 2

In this Example, the activation of an endogenous neural stem cell or progenitor cells by a HGF protein-containing scaffold was evaluated.

### (Material and Method)

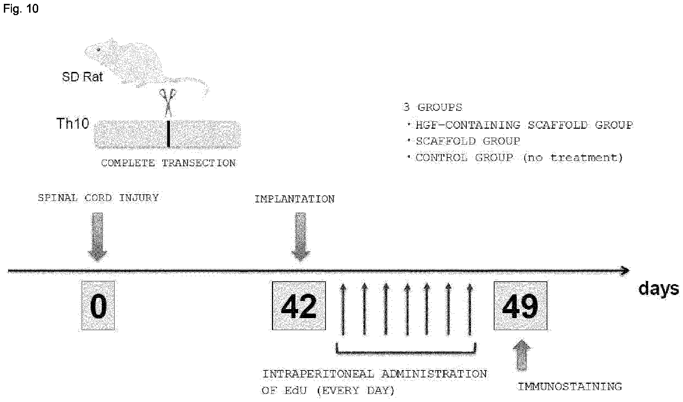

Three groups were prepared in the same manner as in Example 1. Further, immediately after the implantation of the HGF protein-containing sustained release-type collagen gelatin scaffold on the 42nd day, 5-ethynyl-2'-deoxyuridine (EdU) was intraperitoneally administered to perform EdU labeling. A tissue was collected on a 7th day after the implantation, and the tissue was subjected to immunostaining and Western blotting (SOX2, Musashi 1 GFAP, DCX, Hoechst, and Nestin) (see FIG. 10).

References of the respective evaluation methods were as follows: "Chondroitinase and Growth Factors Enhance Activation and Oligodendrocyte Differentiation of Endogenous Neural Precursor Cells after Spinal Cord Injury, 2012," "Cetuximab modified collagen scaffold directs neurogenesis of injury-activated endogenous neural stem cells for acute spinal cord injury repair, 2017," and "Regeneration of the central nervous system using endogenous repair mechanisms, 2007."

### (Evaluation of Activation of Endogenous Neural Stem/Progenitor Cells by HGF Protein-containing Scaffold)

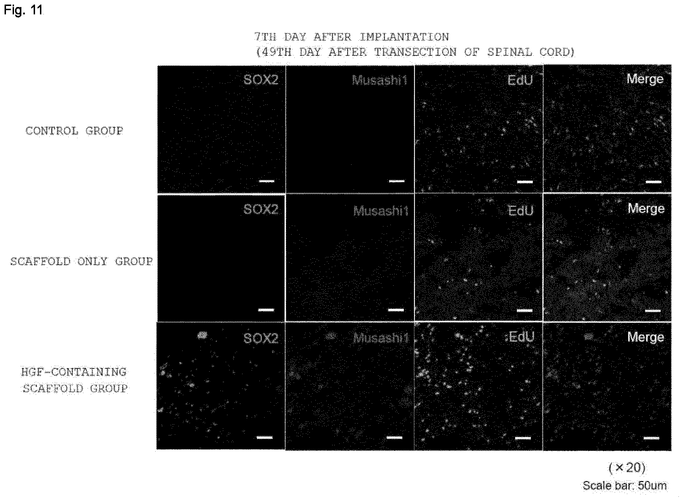

The results of the immunostaining with SOX2, Musashi 1, and EdU are shown in FIG. 11. It was recognized that the numbers of SOX2/EdU co-positive cells, and Musashi 1 positive cells in the HGF protein-containing scaffold group were larger than those in the control groups.

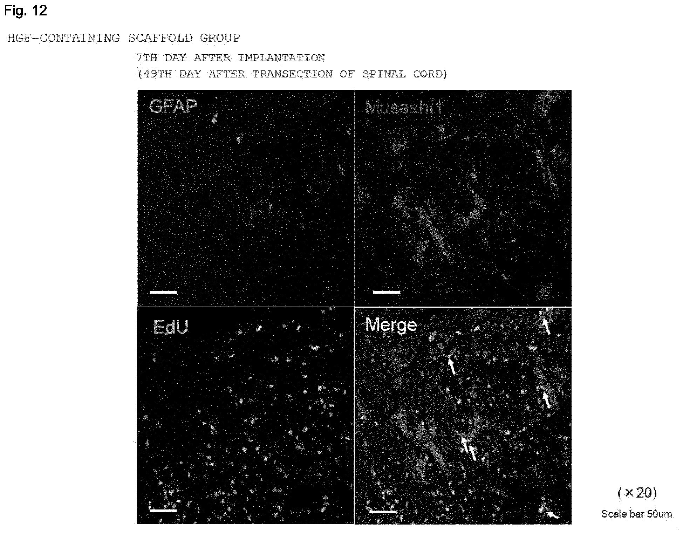

The SOX2/EdU co-positive cells and Musashi 1/EdU co-positive cells in the HGF protein-containing scaffold group are shown in FIG. 12. It was recognized that the HGF protein-containing scaffold group contained large amounts of the SOX2/EdU co-positive cells and the Musashi 1/EdU co-positive cells.

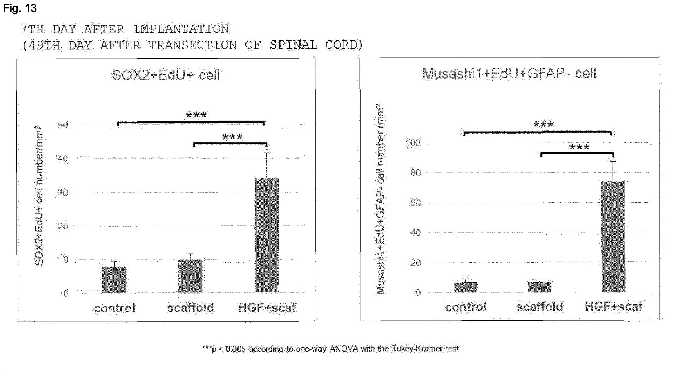

The quantitative evaluation of the activation of the endogenous neural stem/progenitor cells by the HGF protein-containing scaffold is shown in FIG. 13. It was recognized that in the HGF protein-containing scaffold group, the numbers of SOX2-expressing cells, the SOX2/EdU co-positive cells, and the Musashi 1 positive cells in a lesion epicenter were significantly large.

Accordingly, the HGF protein-containing scaffold has a growing effect on the endogenous neural stem/progenitor cells through the sustained release of the HGF protein.

### (Evaluation of Differentiating Action on Endogenous Neural Stem/Progenitor Cell into Neuron exhibited by HGF Protein-containing Scaffold)

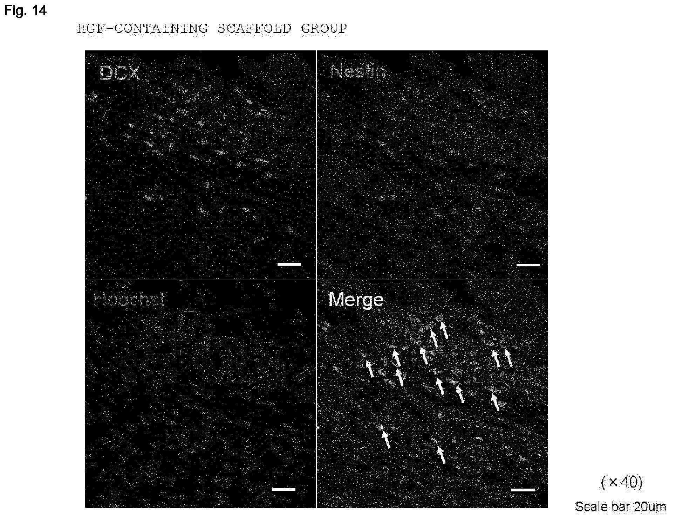

The results of the immunostaining with DCX, Nestin, and Hoechst are shown in FIG. 14. It was recognized that the HGF protein-containing scaffold group contained a large amount of cells (co-positive cells) co-stained with DCX, which was an immature neuron marker, and Nestin, which was an undifferentiated marker.

Accordingly, the HGF protein-containing scaffold has an inducing effect on the differentiation of the endogenous neural stem/progenitor cells into a nerve cell through the sustained release of the HGF protein.

### (General Remarks on Example 2)

It was recognized that the HGF protein-containing scaffold had the following effects:
(1) a growing effect on an endogenous neural stem/progenitor cell in the lesion site of the spinal cord; and
(2) an inducing effect on the differentiation of an endogenous neural stem/progenitor cells into a nerve cell in the lesion site of the spinal cord.

### Example 3

The respective effects of the HGF protein-containing scaffold were recognized by Example 1 and Example 2. Although the alleviation of an inferior spinal cord microenvironment, in which nerve regeneration after chronic complete injury was inhibited, by the HGF protein-containing collagen gelatin scaffold was significantly observed, no improvement in motor function was able to be observed.

The inventors of the present invention have conceived a cause for the fact that no improvement in motor function was achieved as follows: the occurrence of a large cavity in a lesion site resulted in an insufficient number of nerve cells, and hence a sufficient functional improvement was not observed.

In view of the foregoing, in this Example, in addition to the implantation of the HGF protein-containing scaffold in the lesion site, an iPS cell-derived neural stem/progenitor cells was further administered thereto. That is, the agents of the present invention were evaluated.

### (Material and Method)

A complete spinal cord injury model was produced by transecting the high spinal cord of the tenth thoracic vertebra of a female nude rat (rnu/rnu, body weight: from 130 g to 150 g). On a 42nd day after the injury, a HGF protein-containing scaffold (HGF protein-containing sustained release-type collagen gelatin scaffold) was surgically implanted in the model. On a 49th day after the injury, clinical grade iPS cell-derived neural stem/progenitor cells (YZWJs513 hiPSC-derived NS/PCs) were transplanted to 3 sites in each of which the HGF protein-containing scaffold was implanted (TP+Combined group).

In a control group, no treatment was performed on the 42nd day after the injury, and the cells were administered on the 49th day after the injury (TP only group).

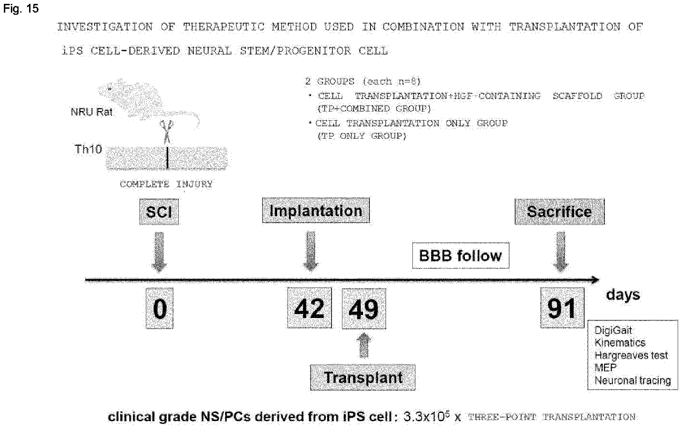

With regard to the administered cells, the YZWJs513 hiPSC-derived NS/PCs were administered in a total number of 1×10⁶ cells to the rostral region, epicenter, and caudal region of the lesion site (three-point administration). After that, the motor evaluation of the hindlimb motor function of the rat and the evaluation of the self-voiding function thereof were performed by using a Basso, Beattie, and Bresnahan (BBB) score every one week. The rat was observed for a time period of up to 91 days after the injury. After that, the spinal cord of the rat was collected and evaluated by immunostaining (see FIG. 15). More specifically, 3.3×10⁵ cells were administered to each point, and hence the total of the cells administered to the 3 points was 1×10⁶ cells (1,000,000 cells).

The used HGF protein-containing scaffold is the same as that of Example 1.

### (Evaluation of Inhibiting Action on Cavitation of Lesion Site of Spinal Cord)

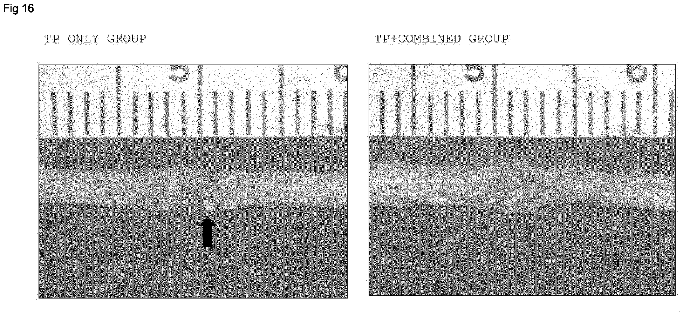

A spinal cord finding on the 42nd day is shown in FIG. 16.

In the TP+Combined group (the agents of the present invention), unlike the TP only group, the transected spinal cords were connected to each other, and no cavity formation was observed.

Accordingly, the agents of the present invention each have the following effect (cavitation-inhibiting effect): the transected spinal cords are connected to each other to fill a cavity site.

### (Evaluation of Survival and Engraftment Rate of administered iPS Cell-derived Neural Stem/Progenitor Cells)

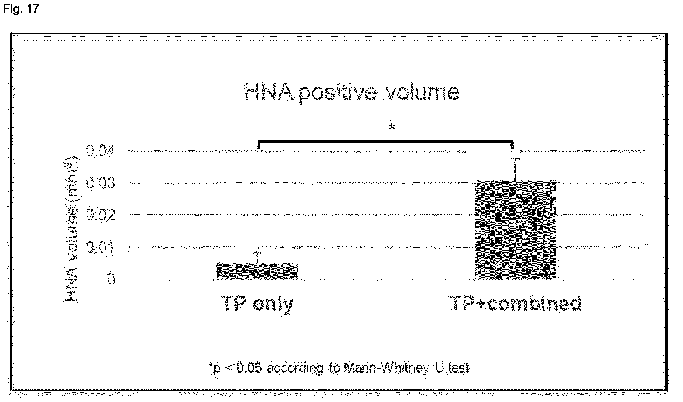

The survival and engraftment rate of the iPS cell-derived neural stem/progenitor cells was measured by using human neutrophil alloantigen (HNA) positive cells as a marker. The results are shown in FIG. 17.

It was recognized that in the TP+Combined group (the agents of the present invention), unlike the TP only group, the survival and engraftment rate of the iPS cell-derived neural stem/progenitor cells significantly increased.

Accordingly, the agents of the present invention each have an increasing effect on the survival and engraftment rate of grafted cells (pluripotent stem cells).

### (Recognition of Differentiation Tendency caused by Grafted Cell)

The differentiation tendency of the iPS cell-derived neural stem/progenitor cells of the TP+Combined group (the agents of the present invention) was compared to that of the TP only group.

The measurement of the percentage of pan-embryonic lethal abnormal vision-like (ELAVL) positive cells (%) and the percentage of glial fibrillary acidic protein (GFAP) positive cells (%) found that in each of both the groups, about 90% of the neural stem/progenitor cells differentiated into neurons and about 5% thereof differentiated into astrocytes (not shown).

The measurement of the percentage of antigen presenting cell (APC) positive cells (%) found that in each of both the groups, about 4% of the neural stem/progenitor cells differentiated into oligodendrocytes (not shown).

The measurement of the percentage of Ki positive cells (%) and the percentage of Nestin positive cells (%) found that in each of both the groups, no tumor growth was observed and the amount of an undifferentiated marker was a low value (not shown).

Thus, it was recognized that the agents of the present invention were each free of an adverse event on a grafted cell (pluripotent stem cell) (in particular, the canceration of the grafted cells).

### (Evaluation of Grafted Cell-derived Nerve Fiber around Lesion Epicenter)

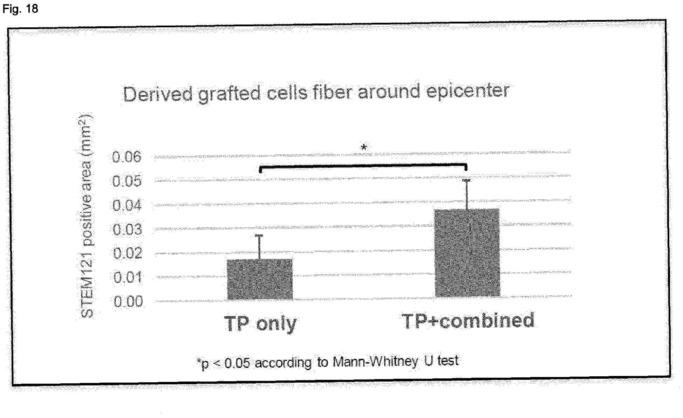

The number of nerve fibers derived from the administered iPS cell-derived neural stem/progenitor cells was measured by using STM121 as a marker. The results are shown in FIG. 18.

It was recognized that in the TP+Combined group (the agents of the present invention), the number of the grafted cell-derived nerve fibers in a lesion epicenter (1 mm) increased as compared to that in the TP only group.

Accordingly, the agents of the present invention each have an increasing effect on the number of the grafted cell-derived nerve fibers.

### (Evaluation of Host-derived Nerve Fiber around Lesion Epicenter)

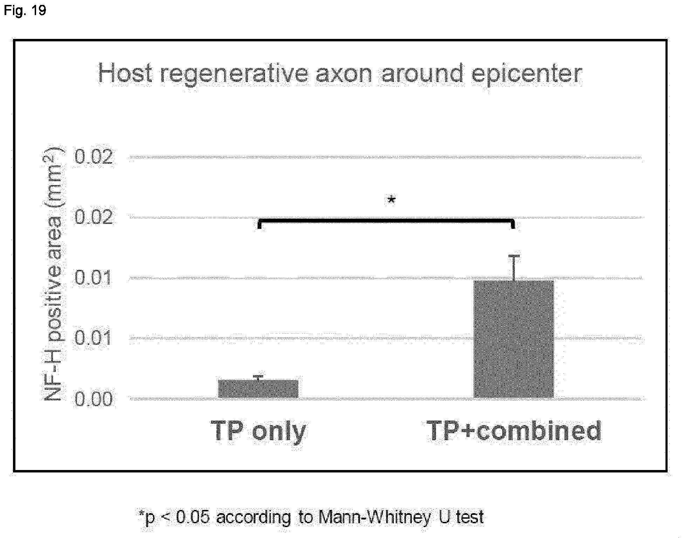

The number of host-derived nerve fibers was measured by using rat neurofilament H (NF-H) as a marker. The results are shown in FIG. 19.

It was recognized that in the TP+Combined group (the agents of the present invention), the number of the host-derived nerve fibers in the lesion epicenter (1 mm) increased as compared to that in the TP only group.

Accordingly, the agents of the present invention each have an increasing effect on the number of the host-derived nerve fibers.

### (Evaluation of Serotonergic Neuron around Lesion Epicenter)

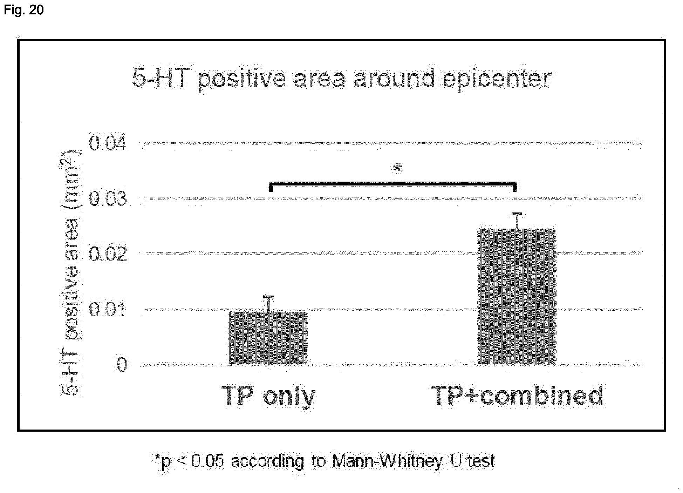

The amount of a serotonergic neuron (5-HT) playing an important role in the recovery of a lower limb motor function after spinal cord injury was measured. The results are shown in FIG. 20.

In the TP only group, the following finding was recognized: nerve fibers that were 5-HT positive in the rostral region of a lesion site died back. Meanwhile, in the TP+Combined group (the agents of the present invention), cells that were 5-HT positive were observed over a range from the rostral region to a lesion epicenter. In addition, in the TP+Combined group, many 5-TH positive fibers were observed at a position caudal to the lesion epicenter by 4 mm.

It was recognized that in the TP+Combination group (the agents of the present invention), the number of the serotonergic neurons in the lesion epicenter (1 mm) increased as compared to that in the TP only group.

Accordingly, the agents of the present invention each have a recovery effect on the lower limb motor function.

### (Evaluation of Newborn Blood Vessel around Lesion Epicenter)

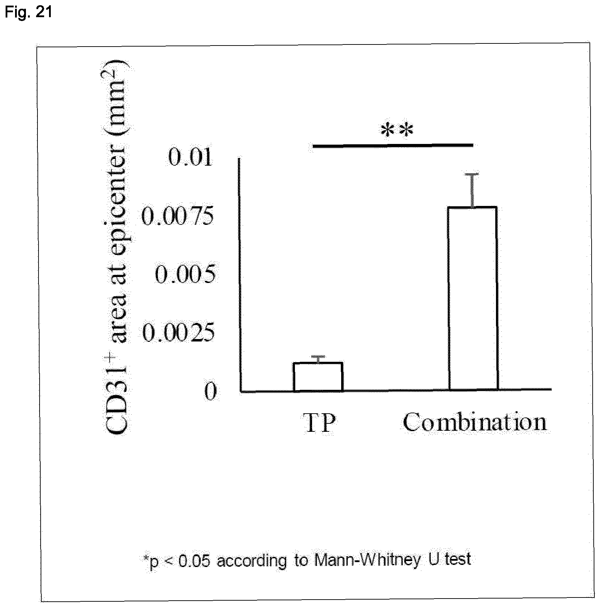

The amount of CD31 that was a vascular endothelial marker for the lesion epicenter was measured. The results are shown in FIG. 21.

It was recognized that in the TP+Combination group (the agents of the present invention), the number of newborn blood vessels in the lesion epicenter increased as compared to that in the TP only group.

Accordingly, the agents of the present invention each have an increasing effect on the number of the newborn blood vessels.

### (Evaluation of Fibrous Scar and Cavity Formation)

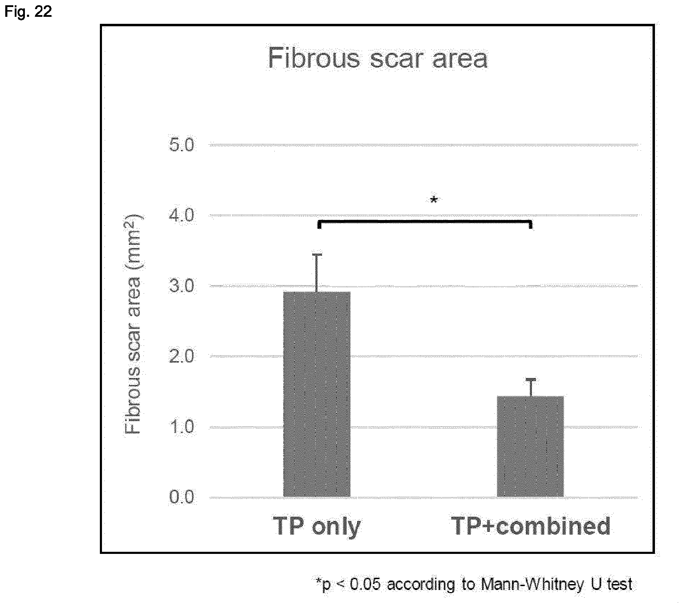

The amount of a fibrous scar was determined by staining the scar with picrosirius with which a collagen fiber was stained red. The results are shown in FIG. 22.

It was recognized that in the TP+Combination group (the agents of the present invention), the number of the fibrous scars significantly reduced as compared to that in the TP only group.

Accordingly, the agents of the present invention each have an inhibiting effect on the fibrous scars and the cavitation.

### (Evaluation of Glial Scar)

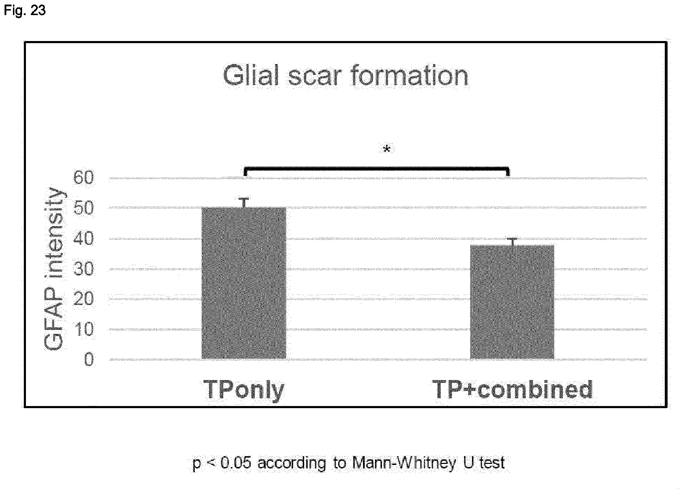

A glial scar was evaluated with the glial fibrillary acidic protein (GFAP) expression intensity of a GFAP positive area around the injury. More specifically, the results of values each obtained by correcting the GFAP expression intensity of the lesion epicenter with a GFAP expression intensity at a position distant from the lesion are shown in FIG. 23.

It was recognized that in the TP+Combination group (the agents of the present invention), a GFAP positive layer was thin and the GFAP expression intensity was significantly low as compared to those in the TP only group.

Accordingly, the agents of the present invention each have an inhibiting effect on the scar formation.

### (Evaluation of Motor Function)

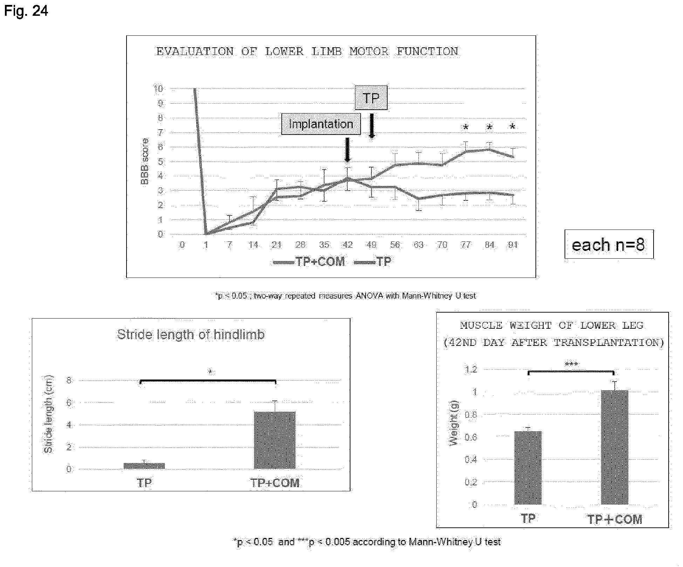

As the evaluation of a motor function (in particular, the evaluation of a lower limb motor function), a Basso-Beattie-Bresnahan (BBB, Basso et al., J. Neurotrauma, vol. 12, pp. 1-21, 1995) score, the stride length of a Digigait small animal gait analysis system, and the muscle weight of a lower leg of the rat were measured. The results are shown in FIG. 24.

Although the BBB score in the TP+Combination group (the agents of the present invention) increased until the 91st day, the score did not increase in the TP only group.

The stride length of a lower limb of the rat in the Digigait small animal gait analysis system significantly increased in the TP+Combination group (the agents of the present invention) as compared to that in the TP only group.

The weight of the triceps muscle of the lower leg significantly increased in the TP+Combination group (the agents of the present invention) as compared to that in the TP only group.

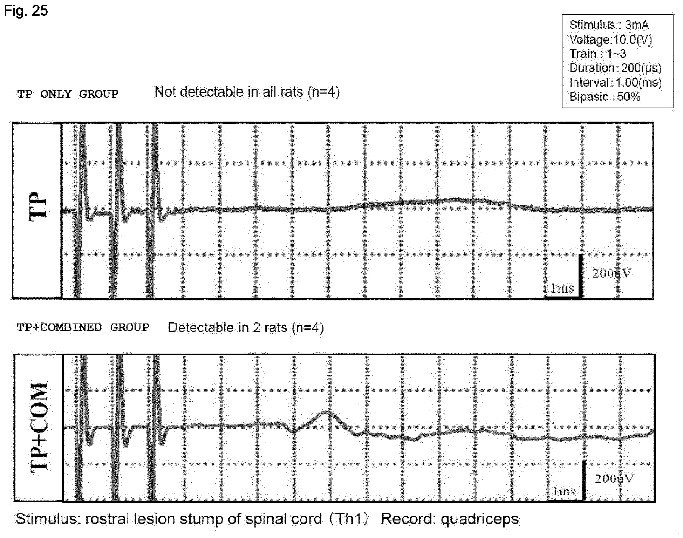

The results of an electrophysiological inspection through use of a transcranial motor evoked potential (MEP) are shown in FIG. 25. More specifically, the spinal cord dura of the rostral region of the lesion of the rat was stimulated, and the MEP was recorded in the quadriceps muscle of a thigh thereof. In the TP+Combination group (the agents of the present invention), unlike the TP only group, a waveform was able to be detected.

Those results showed that the TP+Combination group (the agents of the present invention) was able to improve the motor function of the rat, but the TP only group could not improve the motor function.

Accordingly, the agents of the present invention each have a motor dysfunction-alleviating effect (in particular, a lower limb muscle mass-increasing effect).

### (Evaluation of Voiding Function)

It has been known that the lamina muscularis mucosae of a bladder wall become thicker in spinal cord injury. In view of the foregoing, the voiding function of the rat was observed with a bladder tissue.

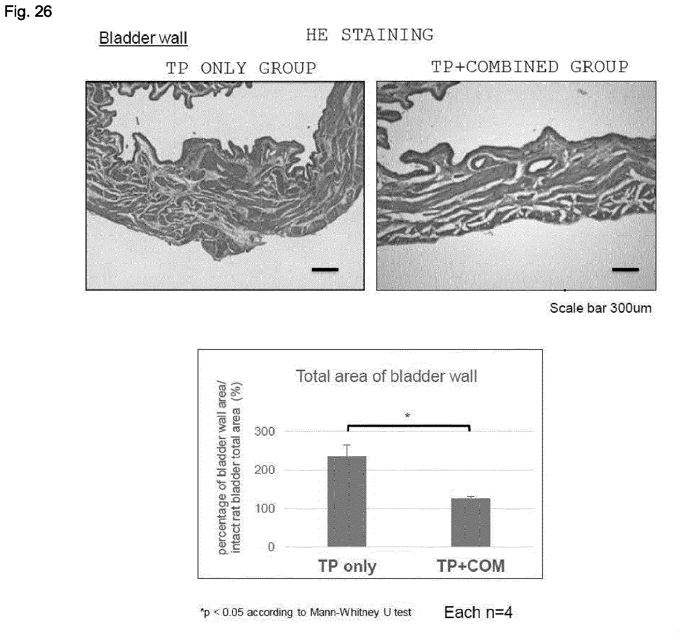

It was recognized that with regard to a HE staining image (the upper part of FIG. 26), the lamina muscularis of the tissue became thinner in the TP+Combination group (the agents of the present invention).

In addition, it was recognized that the area of the entire circumference of the bladder wall corrected with the bladder wall of a normal and same week-old rat that was not subjected to any spinal cord injury (the lower part of FIG. 26) in the TP+Combination group (the agents of the present invention) was smaller than that in the TP only group.

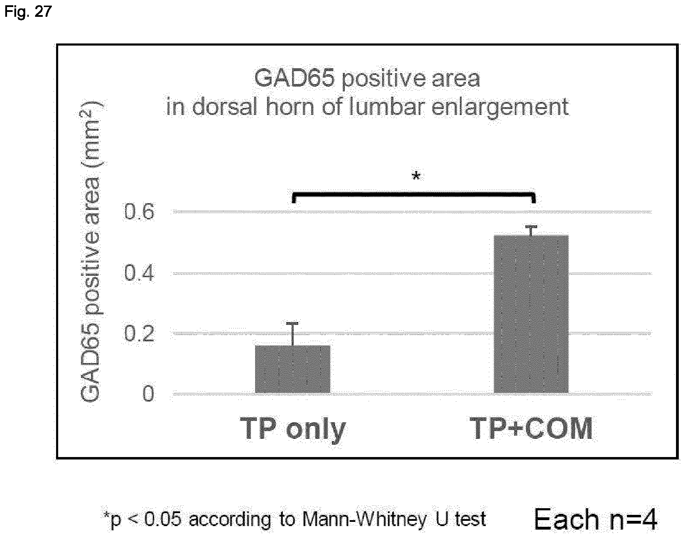

The quantitative evaluation of GAD65, which was a GABAergic neuron marker in a lumbar spinal cord dorsal horn region involved in the voiding function, was performed (FIG. 27). It was recognized that the amount of GAD65 in the TP+Combination group (the agents of the present invention) was larger than that in the TP only group.

It is conceivable from those results that as a result of an increase in inhibitory input in the lumbar spinal cord dorsal horn region, the muscle spasm of the detrusor muscle of the rat was alleviated, and hence the voiding function was improved.

Accordingly, the agents of the present invention each have an alleviating effect on voiding dysfunction.

### (Expression Amount of HGF Protein around Lesion Epicenter)

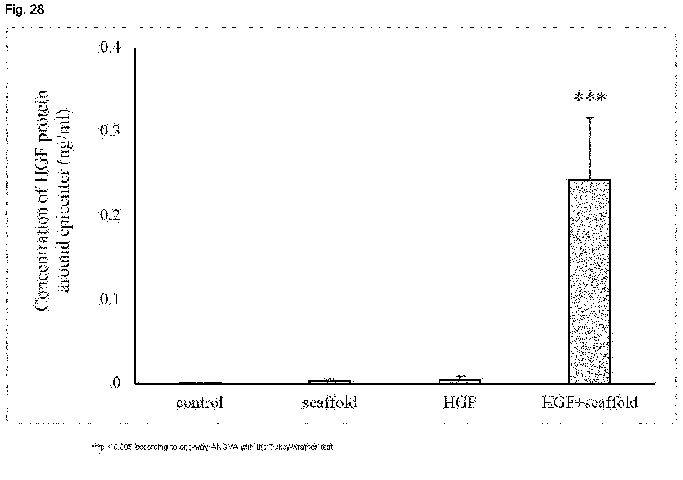

The expression amount of a HGF protein around the lesion epicenter on the 91st day after the injury was measured. The measured results are shown in FIG. 28. It was recognized that in the TP+Combination group (the agents of the present invention: "HGF+scaffold" in the figure), the amount of the HGF protein around the lesion epicenter was larger than that in any other group.

Accordingly, the agents of the present invention each have the effect by which the HGF protein is sustained-released for a long time period.

### (General Remarks on Examples)

It was recognized from the results of Examples 1 to 3 that the agents of the present invention each had the following effects:
(1) an inhibiting effect on spinal cord cavitation in a lesion site of a spinal cord;
(2) an increasing effect on the survival and engraftment rate of grafted cells;
(3) an increasing effect on the number of grafted cell-derived nerve fibers;
(4) an increasing effect on the number of host-derived nerve fibers in the lesion site of the spinal cord;
(5) an increasing effect on the number of newborn blood vessels in the lesion site of the spinal cord;
(6) an inhibiting effect on a fibrous scar in the lesion site of the spinal cord;
(7) an inhibiting effect on scar formation in the lesion site of the spinal cord;
(8) an alleviating effect on motor dysfunction due to spinal cord injury;
(9) an alleviating effect on voiding dysfunction due to the spinal cord injury;
(10) a sustained-releasing effect on a HGF protein in the lesion site of the spinal cord;
(11) an anti-inflammatory action and effect in the lesion site of the spinal cord;
(12) a neuroprotective action and effect in the lesion site of the spinal cord;
(13) a promoting effect on axon extension in the lesion site of the spinal cord (in particular, an increasing effect on the number of regenerated axons and an increasing effect on the density of the regenerated axons);
(14) a growing effect on an endogenous neural stem/progenitor cells in the lesion site of the spinal cord; and
(15) an inducing effect on the differentiation of an endogenous neural stem/progenitor cell into a nerve cell in the lesion site of the spinal cord.

In addition, a substance having a c-Met phosphorylation action comparable to that of the HGF protein may have the same action and effect as those of the HGF protein.

### Industrial Applicability

There can be provided a therapeutic agent for spinal cord injury for chronic incomplete spinal cord injury or chronic complete spinal cord injury.

## Claims

1. A therapeutic agent for treatment of chronic incomplete or chronic complete spinal cord injury, comprising the following:
(1) (a) a hepatocyte growth factor protein or a substance having a c-Met phosphorylation action comparable to that of the hepatocyte growth factor protein, or (b) a gene encoding the hepatocyte growth factor protein or a gene encoding the substance having a c-Met phosphorylation action comparable to that of the hepatocyte growth factor protein;
(2) a carrier configured to carry, and to be capable of sustained-releasing, the (a) or the (b); and
(3) a pluripotent stem cell,
wherein the carrier is implanted in a lesion site of a spinal cord, and the pluripotent stem cells are administered to the carrier after the implantation or to the lesion site of the spinal cord.

2. The therapeutic agent according to claim 1, wherein the pluripotent stem cells are an iPS cell-derived neural stem and/or progenitor cells.

3. The therapeutic agent according to claim 1 or 2, wherein the pluripotent stem cells are administered to the carrier after the implantation.

4. The therapeutic agent according to claim 1, wherein the substance having a c-Met phosphorylation action is a hepatocyte growth factor protein, and the pluripotent stem cells are an iPS cell-derived neural stem and/or progenitor cells.

5. The therapeutic agent according to any one of claims 1 to 4, wherein the treatment is to promote regeneration of an injured spinal cord and/or to alleviate an accessory symptom accompanying the spinal cord injury.

6. The therapeutic agent according to claim 5, wherein the promotion of the regeneration of the injured spinal cord is promotion of axon extension, an increase in number of nerve fibers, neurogenesis of an endogenous stem cell, angiogenesis, inhibition of scar formation, inhibition of inflammation, angiogenesis, and/or inhibition of spinal cord cavitation.

7. The therapeutic agent according to claim 5, wherein the accessory symptom accompanying the injured spinal cord is motor dysfunction and/or voiding dysfunction.

8. The therapeutic agent according to claim 7, wherein the motor dysfunction is motor dysfunction of a lower limb.

9. The therapeutic agent according to claim 7, wherein the motor dysfunction is due to a reduction in muscle mass.

10. An agent for alleviating voiding dysfunction in chronic incomplete spinal cord injury or chronic complete spinal cord injury, comprising the following:
(1) a hepatocyte growth factor protein, and a carrier configured to carry, and to be capable of sustained-releasing, the hepatocyte growth factor protein; and
(2) an iPS cell-derived neural stem and/or progenitor cells,
wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.

11. The therapeutic agent according to claim 10, wherein the iPS cell-derived neural stem and/or progenitor cells are administered to the carrier after the implantation.

12. A therapeutic agent for chronic incomplete or chronic complete spinal cord injury, comprising:
a hepatocyte growth factor protein; and
a carrier configured to carry, and to be capable of sustained-releasing, the hepatocyte growth factor protein,
wherein the carrier carrying the hepatocyte growth factor protein is implanted in a lesion site of a spinal cord, and an iPS cell-derived neural stem and/or progenitor cells are administered to the carrier after the implantation or to the lesion site of the spinal cord.

13. A therapeutic agent for chronic incomplete or chronic complete spinal cord injury, comprising an iPS cell-derived neural stem and/or progenitor cells,
wherein the iPS cell-derived neural stem and/or progenitor cells are administered to a lesion site of a spinal cord or a carrier implanted in the lesion site of the spinal cord, the carrier carrying a hepatocyte growth factor protein.

14. An agent for alleviating motor dysfunction in chronic incomplete spinal cord injury or chronic complete spinal cord injury, comprising the following:
(1) a hepatocyte growth factor protein, and a carrier configured to carry, and to be capable of sustained-releasing, the hepatocyte growth factor protein; and
(2) an iPS cell-derived neural stem and/or progenitor cells,
wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.

15. The agent for alleviating motor dysfunction according to claim 14, wherein the motor dysfunction is motor dysfunction of a lower limb.

16. A lower limb muscle mass-increasing agent in chronic incomplete spinal cord injury or chronic complete spinal cord injury, comprising the following:
(1) a hepatocyte growth factor protein, and a carrier configured to carry, and to be capable of sustained-releasing, the hepatocyte growth factor protein; and
(2) an iPS cell-derived neural stem and/or progenitor cells,
wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.

17. A spinal cord cavitation inhibitor in chronic incomplete spinal cord injury or chronic complete spinal cord injury, comprising the following:
(1) a hepatocyte growth factor protein, and a carrier configured to carry, and to be capable of sustained-releasing, the hepatocyte growth factor protein; and
(2) an iPS cell-derived neural stem and/or progenitor cells,
wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.

18. An anti-inflammatory agent for a lesion site in chronic incomplete spinal cord injury or chronic complete spinal cord injury, comprising the following:
(1) a hepatocyte growth factor protein, and a carrier configured to carry, and to be capable of sustained-releasing, the hepatocyte growth factor protein; and
(2) an iPS cell-derived neural stem and/or progenitor cells,
wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.

19. An angiogenesis promoter for a lesion site in chronic incomplete spinal cord injury or chronic complete spinal cord injury, comprising the following:
(1) a hepatocyte growth factor protein, and a carrier configured to carry, and to be capable of sustained-releasing, the hepatocyte growth factor protein; and
(2) an iPS cell-derived neural stem and/or progenitor cells,
wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.

20. A scar formation inhibitor for a lesion site in chronic incomplete spinal cord injury or chronic complete spinal cord injury, comprising the following:
(1) a hepatocyte growth factor protein, and a carrier configured to carry, and to be capable of sustained-releasing, the hepatocyte growth factor protein; and
(2) an iPS cell-derived neural stem and/or progenitor cells,
wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.

21. An axon extension promoter for a lesion site in chronic incomplete spinal cord injury or chronic complete spinal cord injury, comprising the following:
(1) a hepatocyte growth factor protein, and a carrier configured to carry, and to be capable of sustained-releasing, the hepatocyte growth factor protein; and
(2) an iPS cell-derived neural stem and/or progenitor cells,
wherein the carrier carrying the hepatocyte growth factor protein is implanted in a lesion site of a spinal cord, and the iPS cell-derived neural stem and/or progenitor cells are administered to the carrier after the implantation or to the lesion site of the spinal cord.

22. A therapeutic method for chronic incomplete or chronic complete spinal cord injury, comprising the following steps:
(1) a step of implanting, in a lesion site of a spinal cord of a target having spinal cord injury, a carrier configured to carry, and to be capable of sustained-releasing,
(a) a hepatocyte growth factor protein or a substance having a c-Met phosphorylation action comparable to that of the hepatocyte growth factor protein, or
(b) a gene encoding the hepatocyte growth factor protein or a gene encoding the substance having a c-Met phosphorylation action comparable to that of the hepatocyte growth factor protein; and
(2) a step of administering a pluripotent stem cell to the carrier after the implantation or to the lesion site of the spinal cord.

23. A kit for treating chronic incomplete or chronic complete spinal cord injury, comprising the following:
(1) (a) a hepatocyte growth factor protein or a substance having a c-Met phosphorylation action comparable to that of the hepatocyte growth factor protein, or (b) a gene encoding the hepatocyte growth factor protein or a gene encoding the substance having a c-Met phosphorylation action comparable to that of the hepatocyte growth factor protein;
(2) a carrier configured to carry, and to be capable of sustained-releasing, the (a) or the (b); and
(3) a pluripotent stem cell,
wherein the carrier configured to carry the (a) or the (b) is implanted in a lesion site of a spinal cord of a spinal cord injury patient, and the pluripotent stem cells are administered to the carrier after the implantation or to the lesion site of the spinal cord.
